# EUROPEAN PATENT APPLICATION

(11) **EP 4 177 275 A1**
(43) Date of publication of application: **10.05.2023**
(21) Application number: 21833780.6
(22) Date of filing: 02.07.2021
(51) Int. Cl.: C07K 16/36, C07K 16/46, C12N 15/13, C12N 15/63, A61K 39/395, A61P 7/02

(54) **COAGULATION FACTOR XI (FXI) BINDING PROTEIN**

(30) Priority: 03.07.2020 CN 202010636605; 03.07.2020 CN 202010636876; 03.07.2020 CN 202010637094
(71) Applicant: Suzhou Alphamab Co., Ltd, Jiangsu 215125 (CN)
(72) Inventor: XU, Ting, Suzhou, Jiangsu 215125 (CN); WANG, Xiaoxiao, Suzhou, Jiangsu 215125 (CN); JIN, Yuhao, Suzhou, Jiangsu 215125 (CN); GUO, Kangping, Suzhou, Jiangsu 215125 (CN); WANG, Ling, Suzhou, Jiangsu 215125 (CN); PANG, Minjie, Suzhou, Jiangsu 215125 (CN); WANG, Pilin, Suzhou, Jiangsu 215125 (CN)
(74) Representative: Nederlandsch Octrooibureau
(86) International application number: PCT/CN2021/104173
(87) International publication number: WO 2022/002233

(57) **Abstract**

The present invention relates to the pharmaceutical and biological field, and discloses a single-domain antibody against coagulation factor XI (FXI) and derived protein thereof. In particular, the present invention discloses a coagulation factor XI (FXI) binding protein derived from a single-domain antibody against coagulation factor XI (FXI) and use thereof.

## Description

### TECHNICAL FIELD

The present invention relates to the pharmaceutical and biological field, and discloses a single-domain antibody against coagulation factor XI (FXI) and derived protein thereof. In particular, the present invention discloses a coagulation factor XI (FXI) binding protein derived from a single-domain antibody against coagulation factor XI (FXI) and use thereof.

### BACKGROUND

Coagulation factors are various protein components involved in the process of blood coagulation. Its physiological role is to be activated when a blood vessel bleeds so as to bind platelets and to fill leaks on the blood vessel. This process is called coagulation. They are produced in part by the liver, and can be inhibited by coumarin. For uniform nomenclature, the World Health Organization has numbered the coagulation factors with Roman numerals in the order in which they were discovered, with coagulation factors I, II, III, IV, V, VII, VIII, IX, X, XI, XII, XIII, and the like.

Coagulation factor XI (FXI) is a dimer composed of identical 80 KDa subunits, with each subunit consisting, starting from the N-terminus, of four Apple domains (A1, A2, A3 and A4) and a catalytic domain. FXI is a zymogen that circulates in complex with kininogen (HK) having a high molecular weight. HK binds the A2 domain in FXI and is a physiological cofactor of FXIIa that activates FXI to FXIa. The remaining Apple domains in FXI also mediate important physiological functions. For example, the FIX-binding exosite is located in A3, whereas the FXIIa-binding site is in A4. Residues critical for FXI dimerization are also located in A4.

Studies have shown that FXI plays a key role in the pathological process of thrombosis, contributes relatively little to hemostasis, and is therefore a promising target for thrombus. In a phase II trial with FXI antisense oligonucleotides (ASO) by Ionis Pharmaceuticals Inc. (Buller et al., N Engl J Med 2015, 372:232-240), the patients subjected to total knee arthroplasty had significant venous thromboembolism (VTE) mitigation caused by FXI ASO under the trend of less bleeding compared with enoxaparine. Human genetics and epidemiological studies (Duga et al., Semin Thromb Hemost 2013; Chen et al., Drug Discov Today 2014; Key, Hematology Am Soc Hematol Educ Program 2014; 2014:66-70) indicated that severe FXI deficiency (hemophilia C) reduces the risk of ischemic stroke and deep vein embolism; conversely, increased FXI levels are associated with a higher risk of VTE and ischemic stroke. Furthermore, several preclinical studies have shown that FXIa inhibition or loss of function mediates thrombotic protection without compromising hemostasis (Chen et al., Drug Discov Today 2014). Notably, monoclonal antibodies 14E11 and 1A6 produced significant thrombus mitigation in the baboon AV branch thrombosis model (U.S. Pat. No. 8,388,959; Tucker et al., Blood 2009, 113:936-944; Cheng et al., Blood 2010, 116:3981-3989). Furthermore, 14E11 (in its cross-reaction with mouse FXI) provided protection in a mouse acute ischemic stroke experimental model (Leung et al., Transl Stroke Res 2012, 3:381-389). Additional mAb studies targeting FXI in preclinical models have also been reported which confirm FXI as an anti-thrombotic target with a minimal risk of hemorrhage (van Montfoort et al., Thromb Haemost 2013, 110; Takahashi et al., Thromb Res 2010, 125:464-470; van Montfoort, Ph.D. Thesis, University of Amsterdam, Amsterdam, Netherlands, Nov. 14, 2014). Inhibition of FXI is therefore a promising strategy for novel anti-thrombotic therapy with an improved benefit-risk ratio compared with current standard anticoagulants.

### BRIEF DESCRIPTION OF THE DRAWINGS

FIG. 1 shows the blocking activity of the FXI single-domain antibody-Fc fusion protein against FXI (APTT assay).
FIG. 2 shows the blocking activity of the humanized FXI single-domain antibody-Fc fusion protein against FXI (APTT assay).
FIG. 3 shows the inhibitory effect of the huFE bispecific antibody-Fc fusion protein against human FXI activity.
FIG. 4 shows the inhibitory activity of the huFE bispecific antibody-Fc fusion protein against APTT of human whole plasma.
FIG. 5 shows the inhibitory activity of the huFE bispecific antibody-Fc fusion protein against APTT of monkey whole plasma.
FIG. 6 shows the inhibitory activity of the huFE bispecific antibody-Fc fusion protein against APTT of rabbit whole plasma.
FIG. 7 shows the effect of the FXI single-domain antibody-Fc fusion protein and bispecific antibodies on rabbit venous thrombosis.

### DETAILED DESCRIPTION

### Definitions

Unless otherwise indicated or defined, all terms used herein have the ordinary meaning in the art that would be understood by those skilled in the art. Reference is made, for example, to standard manuals, such as Sambrook et al., "Molecular Cloning: A Laboratory Manual" (2nd edition), Vol. 1-3, Cold Spring Harbor Laboratory Press (1989); Lewin, "Genes IV", Oxford University Press, New York, (1990); and Roitt et al., "Immunology" (2nd edition), Gower Medical Publishing, London, New York (1989), and general prior art cited herein; moreover, unless otherwise indicated, all methods, steps, techniques and operations not specifically recited may be, and have been, performed in a manner known per se to those skilled in the art. Reference is also made, for example, to standard manuals, the general prior art mentioned above and to other references cited therein.

Unless otherwise indicated, the terms "antibody" and "immunoglobulin" used interchangeably herein, whether referring to a heavy-chain antibody or to a conventional 4-chain antibody, are used as a general term to include full length antibodies, individual chains thereof, and all portions, domains or fragments thereof (including, but not limited to, antigen binding domains or fragments, such as VHH domains or VH/VL domains, respectively). In addition, the term "sequence" used herein (e.g., in terms "immunoglobulin sequence", "antibody sequence", "single variable domain sequence", "VHH sequence" or "protein sequence") is generally intended to encompass related amino acid sequences and nucleic acid or nucleotide sequences encoding the sequences, unless further limited interpretation is required herein.

The term "domain" as used herein (of a polypeptide or protein) refers to a folded protein structure that is capable of maintaining its tertiary structure independently of the rest of the protein. In general, a domain is responsible for a single functional property of a protein, and in many cases may be added, removed or transferred to other proteins without loss of functions of the rest of the protein and/or the domain.

The term "immunoglobulin domain" as used herein refers to a globular region of an antibody chain (e.g., a chain of a conventional 4-chain antibody or a chain of a heavy chain antibody) or a polypeptide essentially consisting of such globular regions. The immunoglobulin domain is characterized in that it retains the immunoglobulin folding characteristics of an antibody molecule.

The term "immunoglobulin variable domain" as used herein refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are spaced apart by three "complementarity determining regions" or "CDRs" referred to in the art and hereinafter as "complementarity determining region 1" or "CDR1", "complementarity determining region 2" or "CDR2" and "complementarity determining region 3" or "CDR3". Thus, the general structure or sequence of an immunoglobulin variable domain can be expressed as follows: FR1-CDR1-FR2-CDR2-FR3-CDR3-FR4. The immunoglobulin variable domain endows the antibody with specificity for antigen because of its antigen-binding site.

The term "immunoglobulin single variable domain" as used herein refers to an immunoglobulin variable domain that is capable of specifically binding to an epitope of an antigen without pairing with other immunoglobulin variable domains. An example of an immunoglobulin single variable domain in the meaning of the present invention is a "domain antibody", such as immunoglobulin single variable domains VH and VL (VH domain and VL domain). Another example of an immunoglobulin single variable domain is a "VHH domain" (or abbreviated as "VHH") of the family Camelidae as defined below.

"VHH domain", also known as heavy chain single-domain antibody, VHH, VHH domain, VHH antibody fragment and VHH antibody, is the variable domain of antigen-binding immunoglobulin known as a "heavy chain antibody" (i.e., "an antibody devoid of light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). The term "VHH domain" is used to distinguish the variable domain from the heavy chain variable domain (which is referred to herein as "VH domain") present in the conventional 4-chain antibody and the light chain variable domain (which is referred to herein as a "VL domain") present in the conventional 4-chain antibody. VHH domains specifically bind to an epitope without the need for an additional antigen-binding domain (as opposed to the VH or VL domain in the conventional 4-chain antibody, in which case the epitope is recognized by the VL domain together with the VH domain). The VHH domain is a small, stable and efficient antigen recognition unit formed by a single immunoglobulin domain.

In the context of the present invention, the terms "heavy chain single-domain antibody", "VHH domain", "VHH", "VHH domain", "VHH antibody fragment" and "VHH antibody" can be used interchangeably.

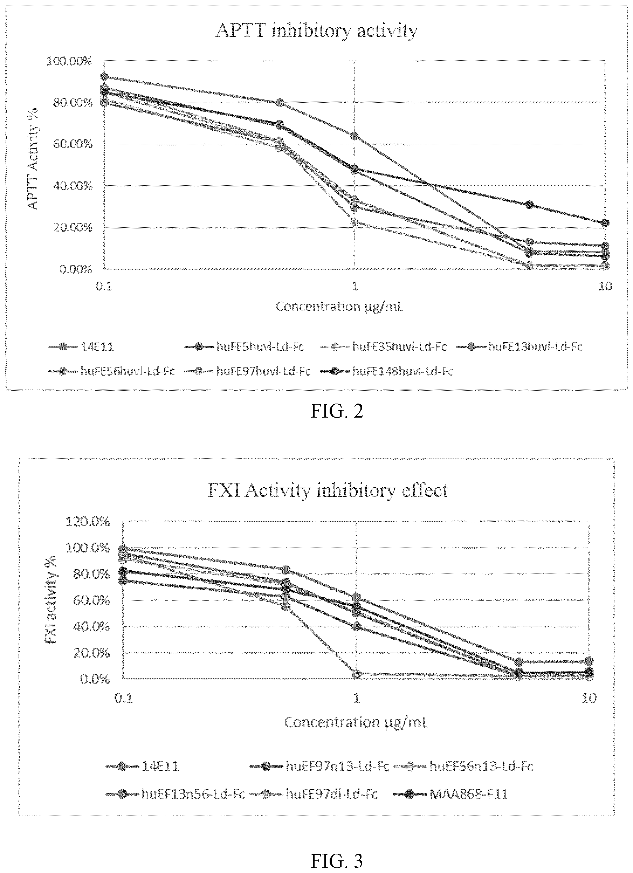

For example, in Riechmann and Muyldermans, J.Immunol. Methods 231, 25-38 (1999), as shown in FIG. 2, the amino acid residues used in VHH domains of the family Camelidae may be numbered according to the general numbering method of VH domains given by Kabat et al. (Kabat et al., Sequences of Proteins of Immunological Interest, 5th Ed. Public Health Service, National Institutes of Health, Bethesda, Md. (1991)).

Alternative methods for numbering amino acid residues of VH domains are known in the art and may also be similarly applied to VHH domains. For example, the Chothia CDR refers to a position of a structural loop (Chothia and Lesk, J. Mol. Biol. 196:901-917 (1987)). The AbM CDR refers to a compromise between Kabat hypervariable region and Chothia structure loop, and is used in Oxford Molecular's AbM antibody modeling software. The "Contact" CDR is based on analysis of available complex crystal structures. The residues of CDR from each method are described below:

| **Ring** | **Kabat** | **AbM** | **Chothia** | **Contact** |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 (Kabat numbering) | H31-H35B | H26-H35B | H26-H32 | H30-H35B |
| HCDR1 (Chothia number) | H31-H35 | H26-H35 | H26-H32 | H30-H35 |
| HCDR2 | H50-H65 | H50-H58 | H53-H55 | H47-H58 |
| HCDR3 | H95-H102 | H95-H102 | H96-H101 | H93-H101 |

The CDR of the antibody may also be IMGT-CDR, a form of CDR definition based on the IMGT antibody code obtained by integrating structural information of over 5000 sequences. In VH CDR encoding of IMGT, the CDR1: 27-38; the CDR2: 56-65; the CDR3: 105-117.

However, it should be noted that, as is well known in the art for VH domains and for VHH domains, the total number of amino acid residues in each CDR may vary and may not correspond to the total number of amino acid residues indicated by the Kabat numbering (that is, one or more positions according to the Kabat numbering may not be occupied in the actual sequence, or the actual sequence may contain more amino acid residues than the number allowed by the Kabat numbering). This means that, generally, the numbering according to Kabat may or may not correspond to the actual numbering of the amino acid residues in the actual sequence.

For example, CDRs may include "extended CDR", such as, 24-36 or 24-34 (LCDR1), 46-56 or 50-56 (LCDR2) and 89-97 or 89-96 (LCDR3) in the VL; 26-35 (HCDR1), 50-65 or 49-65 (HCDR2) and 93-102, 94-102 or 95-102 (HCDR3) in the VH.

The total number of amino acid residues in a VHH domain will usually be in the range of 110 to 120, often between 112 and 115. However, it should be noted that smaller and longer sequences may also be suitable for the purposes described herein.

Other structural and functional properties of VHH domains and polypeptides containing the same can be summarized as follows:
the VHH domain (which has been naturally "designed" to functionally bind to an antigen in the absence of and without interaction with a light chain variable domain) can be used as a single and relatively-small functional antigen binding structural unit, domain or polypeptide. This distinguishes VHH domains from the VH and VL domains of conventional 4-chain antibodies, wherein the VH and VL domains per se are generally not suitable for practical application as single antigen binding proteins or immunoglobulin single variable domains, but need to be combined in a certain form or another form to provide functional antigen binding units (e.g., in the form of a conventional antibody fragment such as a Fab fragment; or in the form of an scFv consisting of a VH domain covalently linked to a VL domain).

Due to these unique properties, compared with conventional VH and VL domains, scFv and conventional antibody fragments (e.g., Fab- or F(ab')₂ fragments), VHH domains, either alone or as part of a larger polypeptide, offer a number of superior significant advantages: only a single domain is required to bind to an antigen with high affinity and high selectivity, so that there is no need to have two separate domains present, nor to assure that these two domains are present in the right spatial conformation and configuration (e.g., the use of specially designed linker is generally required for a scFv); VHH domains can be expressed from a single gene and do not require post-translational folding or modification; VHH domains can be easily engineered into multivalent and multi-specific formats (formatting); VHH domains are highly soluble and do not have a tendency to aggregate; VHH domains are highly stable to heat, pH, proteases and other denaturing agents or conditions and thus, may be prepared, stored or transported without the use of refrigeration equipment, so that the cost, time and environment can be saved; VHH domains are easy to prepare and relatively inexpensive, even on a manufacturing scale; VHH domains are relatively small compared with conventional 4-chain antibodies and antigen-binding fragments thereof (about 15 kDa or 1/10 of conventional IgG in size), and therefore show higher tissue permeability and can be administered at higher doses compared with conventional 4-chain antibodies and antigen-binding fragments thereof; VHH domains can show so-called cavity-binding properties (particularly due to their extended CDR3 loop, compared with conventional VH domains) and can therefore also access targets and epitopes not accessible to conventional 4-chain antibodies and antigen-binding fragments thereof.

Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp. 2645-2652, 17 June, 2009 and WO94/04678. The "humanization" of VHH domains derived from the family Camelidae can be obtained by replacing one or more amino acid residues in the amino acid sequence of the original VHH sequence with one or more amino acid residues present at the corresponding positions in a VH domain of a human conventional 4-chain antibody (also referred to herein as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences. Humanization can be accomplished using methods for protein surface amino acid humanization (resurfacing) and/or humanized CDR grafting to a universal framework, for example, as exemplified in the examples.

As used herein, the term "epitope" or "antigenic determinant" used interchangeably herein refers to any antigenic determinant on an antigen to which the paratope of an antibody binds. The antigenic determinant generally comprises chemically active surface groups of molecules such as amino acids or sugar side chains, and usually has specific three-dimensional structural characteristics and specific charge characteristics. For example, an epitope typically comprises at least 3, 4, 5, 6, 7, 8, 9, 10, 11, 12, 13, 14 or 15 contiguous or non-contiguous amino acids in a unique spatial conformation, and it may be a "linear" epitope or a "conformational" epitope. See, e.g., Epitope Mapping Protocols in Methods in Molecular Biology, volume 66, G.E. Morris, Ed. (1996). In a linear epitope, all points of interaction between a protein and an interacting molecule (e.g., an antibody) exist linearly along the primary amino acid sequence of the protein. In a conformational epitope, the points of interaction exist across amino acid residues on the protein that are separated from one another.

Epitopes of a given antigen can be identified using a number of epitope mapping techniques well known in the art. See, e.g., Epitope Mapping Protocols in Methods in Molecular Biology, volume 66, G.E. Morris, Ed. (1996). For example, a linear epitope can be determined by, for example, the method as follows: a plurality of peptides corresponding to portions of the protein molecule are synthesized simultaneously on a solid support, and these peptides are reacted with the antibody while still attached to the support. Such techniques are known in the art and are described, for example, in U.S. Pat. No. 4,708,871; Geysen et al., (1984) Proc. Natl. Acad. Sci. USA 81:3998-4002; Geysen et al., (1986) Molec. Immunol. 23:709-715. Similarly, conformational epitopes can be identified by determining the spatial configuration of amino acids, for example, by X-ray crystallography and two-dimensional nuclear magnetic resonance. See, e.g., Epitope Mapping Protocols (supra).

Antibodies can be screened for competitively binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Application No. WO03/48731. Therefore, an antibody and an antigen-binding fragment thereof that competes with the antibody molecule of the present invention for binding to the same epitope on FXI can be obtained by conventional techniques known to those skilled in the art.

In general, the term "specificity" refers to the number of different types of antigens or epitopes to which a particular antigen binding molecule or antigen binding protein (e.g., an immunoglobulin single variable domain of the present invention) can bind. Specificity of an antigen binding protein can be determined based on its affinity and/or avidity. Affinity, expressed by a dissociation equilibrium constant (KD) of an antigen to an antigen binding protein, is a measure of the binding strength between an epitope and an antigen binding site on the antigen binding protein: the smaller the KD value, the stronger the binding strength between the epitope and the antigen binding protein (or, the affinity can also be expressed as an association constant (KA), which is 1/KD). As will be appreciated by those skilled in the art, affinity can be determined in a known manner depending on the particular antigen of interest. Avidity is a measure of the binding strength between an antigen binding protein (e.g., an immunoglobulin, an antibody, an immunoglobulin single variable domain, or a polypeptide containing the same) and a related antigen. Avidity is related to both the affinity for its antigen binding site on the antigen binding protein and the number of related binding sites present on the antigen binding protein.

The term "coagulation factor XI (FXI) binding protein" as used herein refers to any protein capable of specifically binding to coagulation factor XI (FXI). The FXI binding protein may include antibodies as defined herein to FXI. The FXI binding protein also encompasses immunoglobulin superfamily antibodies (IgSF) or CDR-grafted molecules.

The "FXI binding protein" of the present invention may comprise at least one immunoglobulin single variable domain (e.g., VHH) that binds to FXI. In some embodiments, the "FXI binding molecule" of the present invention may comprise 2, 3, 4 or more immunoglobulin single variable domains (e.g., VHH) that bind to FXI. The FXI binding protein of the present invention may also comprise, in addition to the immunoglobulin single variable domain binding to FXI, a linker and/or a moiety with effector function, such as a half-life extending moiety (e.g., an immunoglobulin single variable domain that binds to serum albumin), and/or a fusion partner (e.g., serum albumin) and/or a conjugated polymer (e.g., PEG) and/or an Fc region. In some embodiments, the "FXI binding protein" of the present invention also encompasses bispecific antibodies, which contains immunoglobulin single variable domains that bind to different antigens or different regions (e.g., different epitopes) of the same antigen.

Generally, the FXI binding protein of the present invention may bind to an antigen of interest (i.e., FXI) with a dissociation constant (KD) of preferably 10⁻⁷ to 10⁻¹⁰ mol/L (M), more preferably 10⁻⁸ to 10⁻¹⁰ mol/L, even more preferably 10⁻⁹ to 10⁻¹⁰ or less, and/or with an association constant (KA) of at least 10⁷ M⁻¹, preferably at least 10⁸ M⁻¹, more preferably at least 10⁹ M⁻¹ or more preferably at least 10¹⁰ M⁻¹, as measured by Biacore, KinExA or Fortibio assay. Any KD value greater than 10⁻⁴M is generally considered to indicate non-specific binding. Specific binding of an antigen binding protein to an antigen or epitope can be determined in any known suitable way, including, for example, surface plasmon resonance (SPR) assay, Scatchard assay, and/or competitive binding assay (e.g., radioimmunoassay (RIA), enzyme immunoassay (EIA), and sandwich competitive assay) described herein.

Amino acid residues will be represented according to the standard three-letter or one-letter amino acid code as well known and agreed upon in the art. When two amino acid sequences are compared, the term "amino acid difference" refers to the insertion, deletion or substitution of a specified number of amino acid residues at a certain position of the reference sequence as compared with another sequence. In the case of a substitution, the substitution will preferably be a conservative amino acid substitution which means that an amino acid residue is replaced with another amino acid residue that is chemically similar in structure and that has little or no effect on the function, activity or other biological properties of the polypeptide. Such conservative amino acid substitutions are well known in the art, for example conservative amino acid substitutions are preferably made where one amino acid within the following groups (i)-(v) is substituted with another amino acid residue within the same group: (i) smaller aliphatic nonpolar or weakly polar residues: Ala, Ser, Thr, Pro and Gly; (ii) polar negatively-charged residues and their (uncharged) amides: Asp, Asn, Glu and Gln; (iii) polar positively-charged residues: His, Arg and Lys; (iv) larger aliphatic nonpolar residues: Met, Leu, Ile, Val and Cys; and (v) aromatic residues: Phe, Tyr and Trp. Particularly preferred conservative amino acid substitutions are as follows: Ala is substituted with Gly or Ser; Arg is substituted with Lys; Asn is substituted with Gin or His; Asp is substituted with Glu; Cys is substituted with Ser; Gln is substituted with Asn; Glu is substituted with Asp; Gly is substituted with Ala or Pro; His is substituted with Asn or Gln; Ile is substituted with Leu or Val; Leu is substituted with Ile or Val; Lys is substituted with Arg, Gln or Glu; Met is substituted with Leu, Tyr or Ile; Phe is substituted with Met, Leu or Tyr; Ser is substituted with Thr; Thr is substituted with Ser; Trp is substituted with Tyr; Tyr is substituted with Trp or Phe; Val is substituted with Ile or Leu.

"Sequence identity" between two polypeptide sequences indicates the percentage of identical amino acids between the sequences. "Sequence similarity" indicates the percentage of amino acids that are identical or represent conservative amino acid substitutions. Methods for assessing the degree of sequence identity between amino acids or nucleotides are known to those skilled in the art. For example, amino acid sequence identity is typically measured using sequence analysis software. For example, the BLAST program from the NCBI database can be used to determine the identity. For the determination of sequence identity, see, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987 and Sequence Analysis Primer, Gribskov, M. and Devereux, J., eds., M Stockton Press, New York, 1991.

Compared with the natural biological source of a polypeptide or nucleic acid molecule and/or the reaction medium or culture medium used for obtaining the polypeptide or nucleic acid molecule, when the polypeptide or nucleic acid molecule has been separated from at least one other usually associated component (such as another protein/polypeptide, another nucleic acid, another biological component or macromolecule or at least one pollutant, impurity or trace component) in the source or medium (culture medium), the polypeptide or nucleic acid molecule is regarded as "isolated". In particular, a polypeptide or nucleic acid molecule is considered "isolated" when it has been purified at least 2-fold, in particular at least 10-fold, more in particular at least 100-fold and up to 1000-fold or more than 1000-fold. The "isolated" polypeptide or nucleic acid molecule is preferably substantially homogeneous, as determined by suitable techniques (e.g., suitable chromatographic techniques, such as polyacrylamide gel electrophoresis).

The "effective amount" refers to the amount of an FXI binding protein or a pharmaceutical composition of the present invention that results in a reduction in the severity of disease symptoms, an increase in the frequency and duration of asymptomatic period of the disease, or prevention of injury or disability resulting from the affliction of the disease.

As used herein, "thrombosis" refers to the formation or presence of a clot (also referred to as "thrombus") within a blood vessel, thereby impeding the flow of blood through the circulatory system. Thrombosis is typically caused by abnormalities in the composition of the blood, the quality of the vessel wall, and/or the nature of the blood flow. The formation of a clot is typically caused by injury to the vessel wall (such as damage to the vessel wall from trauma or infection) and the slowing or cessation of blood flow through the injury site. In some cases, coagulation abnormalities cause thrombosis.

As used herein, "without compromising hemostasis" means that little or no detectable bleeding is observed in a subject following administration of the FXI binding protein or the pharmaceutical composition of the present invention to the subject. In the case of targeting FXI, inhibition of conversion of FXI into FXIa or inhibition of FXIIa activation to FIX results in the inhibition of coagulation and related thrombosis without bleeding.

The term "subject" as used herein refers to a mammal, particularly a primate, more particularly a human.

### FXI binding protein of the present invention

In one aspect, the present invention provides an FXI binding protein comprising at least one immunoglobulin single variable domain capable of specifically binding to FXI.

In some embodiments, the at least one immunoglobulin single variable domain comprises CDR1, CDR2 and CDR3 of a VHH set forth in any one of SEQ ID NOs: 1-23. The CDRs may be Kabat CDRs, AbM CDRs, Chothia CDRs or IMGT CDRs.

In some embodiments, the at least one immunoglobulin single variable domain comprises a set of CDR1, CDR2 and CDR3 selected from the following sequences:

| **Antibody strain No.** | **CDR1-3 according to Kabat** | **CDR1-3 according to AbM** | **CDR1-3 according to Chothia** | **CDR1-3 according to IMGT** |
|---|---|---|---|---|
| **5** | SEQ ID NO:24-26 | SEQ ID NO:27-29 | SEQ ID NO:30-32 | SEQ ID NO:33-35 |
| 7 | SEQ ID NO:36-38 | SEQ ID NO:39-41 | SEQ ID NO:42-44 | SEQ ID NO:45-47 |
| 11 | SEQ ID NO:48-50 | SEQ ID NO:51-53 | SEQ ID NO:54-56 | SEQ ID NO:57-59 |
| **13** | SEQ ID NO:60-62 | SEQ ID NO:63-65 | SEQ ID NO:66-68 | SEQ ID NO:69-71 |
| 15 | SEQ ID NO:72-74 | SEQ ID NO:75-77 | SEQ ID NO:78-80 | SEQ ID NO:81-83 |
| 17 | SEQ ID NO:84-86 | SEQ ID NO:87-89 | SEQ ID NO:90-92 | SEQ ID NO:93-95 |
| 22 | SEQ ID NO:96-98 | SEQ ID NO:99-101 | SEQ ID NO:102-104 | SEQ ID NO:105-107 |
| 29 | SEQ ID NO:108-110 | SEQ ID NO:111-113 | SEQ ID NO:114-116 | SEQ ID NO:117-119 |
| **30** | SEQ ID NO:120-122 | SEQ ID NO:123-125 | SEQ ID NO:126-128 | SEQ ID NO:129-131 |
| **35** | SEQ ID NO:132-134 | SEQ ID NO:135-137 | SEQ ID NO:138-140 | SEQ ID NO:141-143 |
| 43 | SEQ ID NO:144-146 | SEQ ID NO:147-149 | SEQ ID NO:150-152 | SEQ ID NO:153-155 |
| 49 | SEQ ID NO:156-158 | SEQ ID NO:159-161 | SEQ ID NO:162-164 | SEQ ID NO:165-167 |
| 50 | SEQ ID NO:168-170 | SEQ ID NO:171-173 | SEQ ID NO:174-176 | SEQ ID NO:177-179 |
| **56** | SEQ ID NO:180-182 | SEQ ID NO:183-185 | SEQ ID NO:186-188 | SEQ ID NO:189-191 |
| 70 | SEQ ID NO:192-194 | SEQ ID NO:195-197 | SEQ ID NO:198-200 | SEQ ID NO:201-203 |
| 96 | SEQ ID NO:204-206 | SEQ ID NO:207-209 | SEQ ID NO:210-212 | SEQ ID NO:213-215 |
| **97** | SEQ ID NO:216-218 | SEQ ID NO:219-221 | SEQ ID NO:222-224 | SEQ ID NO:225-227 |
| 107 | SEQ ID NO:228-230 | SEQ ID NO:231-233 | SEQ ID NO:234-236 | SEQ ID NO:237-239 |
| 128 | SEQ ID NO:240-242 | SEQ ID NO:243-245 | SEQ ID NO:246-248 | SEQ ID NO:249-251 |
| **148** | SEQ ID NO:252-254 | SEQ ID NO:255-257 | SEQ ID NO:258-260 | SEQ ID NO:261-263 |
| 163 | SEQ ID NO:264-266 | SEQ ID NO:267-269 | SEQ ID NO:270-272 | SEQ ID NO:273-275 |
| 166 | SEQ ID NO:276-278 | SEQ ID NO:279-281 | SEQ ID NO:282-284 | SEQ ID NO:285-287 |
| 168 | SEQ ID NO:288-290 | SEQ ID NO:291-293 | SEQ ID NO:294-296 | SEQ ID NO:297-299 |

In some embodiments, at least one immunoglobulin single variable domain in the FXI binding protein of the present invention is a VHH. In some embodiments, the VHH comprises an amino acid sequence of any one of SEQ ID NOs: 1-23.

In some embodiments, at least one immunoglobulin single variable domain in the FXI binding protein of the present invention is a humanized VHH.

In some embodiments, at least one immunoglobulin single variable domain in the FXI binding protein of the present invention is a humanized VHH comprising an amino acid sequence having at least 80%, preferably at least 90%, more preferably at least 95%, even more preferably at least 99% sequence identity to any one of SEQ ID NOs: 1-23. In some embodiments, the amino acid sequence of the humanized VHH comprises one or more amino acid substitutions, preferably conservative amino acid substitutions, as compared with any one of SEQ ID NOs: 1-23. For example, 1, 2, 3, 4, 5, 6, 7, 8, 9 or 10 conservative amino acid substitutions are comprised.

In some embodiments, at least one immunoglobulin single variable domain in the FXI binding protein of the present invention is a humanized VHH, wherein the humanized VHH comprises an amino acid sequence of any one of SEQ ID NOs: 300-335.

In some embodiments, the at least one immunoglobulin single variable domain binds to an epitope within the Apple2 domain of FXI. An exemplary amino acid sequence of the Apple2 domain of FXI is set forth in SEQ ID NO: 338. For example, the immunoglobulin single variable domain comprises CDR1, CDR2 and CDR3 of a VHH set forth in any one of SEQ ID NO: 4, SEQ ID NO: 10 or SEQ ID NO: 14. In some embodiments, the immunoglobulin single variable domain comprises a set of CDR1, CDR2 and CDR3 selected from SEQ ID NOs: 60-62, SEQ ID NOs: 63-65, SEQ ID NOs: 66-68, SEQ ID NOs: 69-71, SEQ ID NOs: 132-134, SEQ ID NOs: 135-137, SEQ ID NOs: 138-140, SEQ ID NOs: 141-143, SEQ ID NOs: 180-182, SEQ ID NOs: 183-185, SEQ ID NOs: 186-188 and SEQ ID NOs: 189-191. In some specific embodiments, the immunoglobulin single variable domain comprises an amino acid sequence set forth in any one of SEQ ID NO: 4, SEQ ID NO: 10 or SEQ ID NO: 14. In some specific embodiments, the immunoglobulin single variable domain comprises an amino acid sequence set forth in any one of SEQ ID NOs: 306-323.

In some embodiments, the at least one immunoglobulin single variable domain does not bind to an epitope within the Apple2 domain of FXI. In some embodiments, the at least one immunoglobulin single variable domain does not bind to the Apple2 domain of FXI. In some embodiments, the at least one immunoglobulin single variable domain does not bind to an isolated Apple2 domain polypeptide of FXI.

In some embodiments, the at least one immunoglobulin single variable domain binds to an epitope within the Apple3 domain of FXI. An exemplary amino acid sequence of the Apple3 domain of FXI is set forth in SEQ ID NO: 339. For example, the immunoglobulin single variable domain comprises CDR1, CDR2 and CDR3 of the VHH set forth in SEQ ID NO: 17. In some embodiments, the immunoglobulin single variable domain comprises a set of CDR1, CDR2 and CDR3 selected from SEQ ID NOs: 216-218, SEQ ID NOs: 219-221, SEQ ID NOs: 222-224 and SEQ ID NOs: 225-227. In some specific embodiments, the immunoglobulin single variable domain comprises an amino acid sequence set forth in SEQ ID NO: 17. In some specific embodiments, the immunoglobulin single variable domain comprises an amino acid sequence set forth in any one of SEQ ID NOs: 324-329.

In some embodiments, the at least one immunoglobulin single variable domain binds to an epitope within the Apple4 domain of FXI. An exemplary amino acid sequence of the Apple4 domain of FXI is set forth in SEQ ID NO: 340. For example, the immunoglobulin single variable domain comprises CDR1, CDR2 and CDR3 of the VHH set forth in SEQ ID NO: 1. In some embodiments, the immunoglobulin single variable domain comprises a set of CDR1, CDR2 and CDR3 selected from SEQ ID NOs: 24-26, SEQ ID NOs: 27-29, SEQ ID NOs: 30-32 and SEQ ID NOs: 33-35. In some specific embodiments, the immunoglobulin single variable domain comprises an amino acid sequence set forth in SEQ ID NO: 1. In some specific embodiments, the immunoglobulin single variable domain comprises an amino acid sequence set forth in any one of SEQ ID NOs: 300-305.

In some embodiments, the at least one immunoglobulin single variable domain binds to an epitope within the Apple1-2 region of FXI (a region between the Apple1 domain and the Apple2 domain). An exemplary amino acid sequence of the Apple1-2 region of FXI is set forth in SEQ ID NO: 341.

In some embodiments, the at least one immunoglobulin single variable domain binds to an epitope within the Apple2-3 region of FXI (a region between the Apple2 domain and the Apple3 domain). An exemplary amino acid sequence of the Apple2-3 region of FXI is set forth in SEQ ID NO: 342. For example, the immunoglobulin single variable domain comprises CDR1, CDR2 and CDR3 of the VHH set forth in SEQ ID NO: 20. In some embodiments, the immunoglobulin single variable domain comprises a set of CDR1, CDR2 and CDR3 selected from SEQ ID NOs: 252-254, SEQ ID NOs: 255-257, SEQ ID NOs: 258-260 and SEQ ID NOs: 261-263. In some specific embodiments, the immunoglobulin single variable domain comprises an amino acid sequence set forth in SEQ ID NO: 20. In some specific embodiments, the immunoglobulin single variable domain comprises an amino acid sequence set forth in any one of SEQ ID NOs: 330-335.

In some embodiments, the at least one immunoglobulin single variable domain binds to an epitope within the Apple3-4 region of FXI (a region between the Apple3 domain and the Apple4 domain). An exemplary amino acid sequence of the Apple3-4 region of FXI is set forth in SEQ ID NO: 343.

In some embodiments, the FXI binding protein comprises an immunoglobulin single variable domain that specifically binds to FXI.

In some embodiments, the FXI binding protein comprises at least two, e.g., 2, 3, 4 or more immunoglobulin single variable domains that specifically bind to FXI.

In some embodiments, the at least two immunoglobulin single variable domains bind to or compete for binding to or partially compete for binding to the same region or epitope of FXI, e.g., the at least two immunoglobulin single variable domains are identical.

In some embodiments, the at least two immunoglobulin single variable domains bind to different regions or epitopes of FXI, or do not compete for binding to the same region or epitope of FXI.

Whether two antibodies or immunoglobulin single variable domains bind to or compete for binding to the same region or epitope can be determined through epitope binning by the bio-layer interferometry (BLI), as exemplified in the examples herein.

In some embodiments, the at least two immunoglobulin single variable domains that specifically bind to FXI are directly linked to each other.

In some embodiments, the at least two immunoglobulin single variable domains that specifically bind to FXI are linked to each other via a linker. The linker may be a non-functional amino acid sequence having 1-20 or more amino acids in length and no secondary or higher structure. For example, the linker is a flexible linker, such as GGGGS, GS, GAP and (GGGGS) x 3.

In some embodiments, the FXI binding protein comprises a first immunoglobulin single variable domain capable of specifically binding to FXI and a second immunoglobulin single variable domain capable of specifically binding to FXI, wherein the first immunoglobulin single variable domain and the second immunoglobulin single variable domain bind to different epitopes on FXI.

In some embodiments, the first immunoglobulin single variable domain binds to an epitope within the Apple2 domain of FXI, and the second immunoglobulin single variable domain binds to an epitope within the Apple3 domain of FXI; or
the first immunoglobulin single variable domain binds to an epitope within the Apple2 domain of FXI, and the second immunoglobulin single variable domain binds to an epitope within the Apple4 domain of FXI; or
the first immunoglobulin single variable domain binds to an epitope within the Apple2 domain of FXI, and the second immunoglobulin single variable domain binds to an epitope within the Apple2-3 region of FXI; or
the first immunoglobulin single variable domain binds to an epitope within the Apple3 domain of FXI, and the second immunoglobulin single variable domain binds to an epitope within the Apple4 domain of FXI; or
the first immunoglobulin single variable domain binds to an epitope within the Apple3 domain of FXI, and the second immunoglobulin single variable domain binds to an epitope within the Apple2-3 region of FXI; or
the first immunoglobulin single variable domain binds to an epitope within the Apple4 domain of FXI, and the second immunoglobulin single variable domain binds to an epitope within the Apple2-3 region of FXI.

In some embodiments, the FXI binding protein comprises the first immunoglobulin single variable domain and the second immunoglobulin single variable domain, wherein the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 4; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 9; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 10; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 9; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 10; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 10; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 10, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 10, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 10, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 14, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 14, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 17, and the second immunoglobulin single variable domain comprises CDR1, CDR2 and CDR3 of a VHH set forth in SEQ ID NO: 20.

In some embodiments, wherein the CDR1, CDR2 and CDR3 of the VHH set forth in SEQ ID NO: 1, 4, 9, 10, 14, 17 or 20 are shown in the following table:

| **VHH sequence** | **CDR1-3 according to Kabat** | **CDR1-3 according to AbM** | **CDR1-3 according to Chothia** | **CDR1-3 according to IMGT** |
|---|---|---|---|---|
| **SEQ ID NO:1** | SEQ ID NO:24-26 | SEQ ID NO:27-29 | SEQ ID NO:30-32 | SEQ ID NO:33-35 |
| **SEQ ID NO:4** | SEQ ID NO:60-62 | SEQ ID NO:63-65 | SEQ ID NO:66-68 | SEQ ID NO:69-71 |
| **SEQ ID NO:9** | SEQ ID NO:120-122 | SEQ ID NO:123-125 | SEQ ID NO:126-128 | SEQ ID NO:129-131 |
| **SEQ ID NO:10** | SEQ ID NO:132-134 | SEQ ID NO:135-137 | SEQ ID NO:138-140 | SEQ ID NO:141-143 |
| **SEQ ID NO:14** | SEQ ID NO:180-182 | SEQ ID NO:183-185 | SEQ ID NO:186-188 | SEQ ID NO:189-191 |
| **SEQ ID NO:17** | SEQ ID NO:216-218 | SEQ ID NO:219-221 | SEQ ID NO:222-224 | SEQ ID NO:225-227 |
| **SEQ ID NO:20** | SEQ ID NO:252-254 | SEQ ID NO:255-257 | SEQ ID NO:258-260 | SEQ ID NO:261-263 |

In some embodiments, the FXI binding protein comprises the first immunoglobulin single variable domain and the second immunoglobulin single variable domain, wherein
The first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 4, 306-311; or
the first immunoglobulin single variable domain comprises an amino acid sequence set forth in any one of SEQ ID NOs: 1, 300-305, and the second immunoglobulin single variable domain comprises an amino acid sequence set forth in SEQ ID NO: 9; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 10, 312-317; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 14, 318-323; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 17, 324-329; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 4, 306-311, and the second immunoglobulin single variable domain comprises an amino acid sequence set forth in SEQ ID NO: 9; or
the first immunoglobulin single variable domain comprises an amino acid sequence set forth in any one of SEQ ID NOs: 4, 306-311, and the second immunoglobulin single variable domain comprises an amino acid sequence of a VHH set forth in any one of SEQ ID NOs: 10, 312-317; or
the first immunoglobulin single variable domain comprises an amino acid sequence set forth in any one of SEQ ID NOs: 4, 306-311, and the second immunoglobulin single variable domain comprises an amino acid sequence set forth in any one of SEQ ID NOs: 14, 318-323; or the first immunoglobulin single variable domain comprises an amino acid sequence set forth in any one of SEQ ID NOs: 4, 306-311, and the second immunoglobulin single variable domain comprises an amino acid sequence set forth in any one of SEQ ID NOs: 17, 324-329; or the first immunoglobulin single variable domain comprises an amino acid sequence set forth in any one of SEQ ID NOs: 4, 306-311, and the second immunoglobulin single variable domain comprises an amino acid sequence set forth in any one of SEQ ID NOs: 20, 330-335; or
the first immunoglobulin single variable domain comprises an amino acid sequence set forth in SEQ ID NO: 9, and the second immunoglobulin single variable domain comprises an amino acid sequence set forth in any one of SEQ ID NOs: 10, 312-317; or
the first immunoglobulin single variable domain comprises an amino acid sequence set forth in SEQ ID NO: 9, and the second immunoglobulin single variable domain comprises an amino acid sequence set forth in any one of SEQ ID NOs: 14, 318-323; or
the first immunoglobulin single variable domain comprises an amino acid sequence set forth in SEQ ID NO: 9, and the second immunoglobulin single variable domain comprises an amino acid sequence set forth in any one of SEQ ID NOs: 17, 324-329; or
the first immunoglobulin single variable domain comprises an amino acid sequence set forth in SEQ ID NO: 9, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 10, 312-317, and the second immunoglobulin single variable domain comprises an amino acid sequence set forth in any one of SEQ ID NOs: 14, 318-323; or
the first immunoglobulin single variable domain comprises an amino acid sequence set forth in any one of SEQ ID NOs: 10, 312-317, and the second immunoglobulin single variable domain comprises an amino acid sequence set forth in any one of SEQ ID NOs: 17, 324-329; or
the first immunoglobulin single variable domain comprises an amino acid sequence set forth in any one of SEQ ID NOs: 10, 312-317, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 14, 318-323, and the second immunoglobulin single variable domain comprises an amino acid sequence set forth in any one of SEQ ID NOs: 17, 324-329; or
the first immunoglobulin single variable domain comprises an amino acid sequence set forth in any one of SEQ ID NOs: 14, 318-323, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 17, 324-329, and the second immunoglobulin single variable domain comprises an amino acid sequence set forth in any one of SEQ ID NOs: 20, 330-335.

In some embodiments, wherein the first immunoglobulin single variable domain is located at the N-terminus of the second immunoglobulin single variable domain. In other embodiments, wherein the second immunoglobulin single variable domain is located at the N-terminus of the first immunoglobulin single variable domain.

In some embodiments, the FXI binding protein of the present invention comprises an immunoglobulin Fc region in addition to at least one immunoglobulin single variable domain capable of specifically binding to FXI. The inclusion of an immunoglobulin Fc region in the FXI binding protein of the present invention may allow the binding molecules to form a dimer. The Fc regions useful in the present invention may be from different subtypes of immunoglobulins, for example, IgG (e.g., IgG1, IgG2, IgG3 or IgG4 subtype), IgA1, IgA2, IgD, IgE or IgM.

In some embodiments, mutations may be introduced into the wild-type Fc sequence for altering Fc-mediated related activities. Such mutations include, but are not limited to: a). mutations that alter Fc-mediated CDC activity; b). mutations that alter Fc-mediated ADCC activity; or c). mutations that alter FcRn-mediated half-life *in vivo.* Such mutations are described in the following documents: Leonard G Presta, Current Opinion in Immunology 2008, 20:460-470; Esohe E. Idusogie et al., J Immunol 2000, 164: 4178-4184; RAPHAEL A. CLYNES et al., Nature Medicine, 2000, Volume 6, Number 4: 443-446; Paul R. Hinton et al., J Immunol, 2006, 176:346-356. For example, mutations on 1, 2, 3, 4, 5, 6, 7, 8, 9 or 10 amino acids of the CH2 region may be used to increase or remove Fc-mediated ADCC or CDC activity or to enhance or reduce FcRn affinity. In addition, the stability of the protein may be increased by mutating 1, 2, 3, 4, 5, 6, 7, 8, 9 or 10 amino acids of the hinge region.

In some embodiments, mutations may be introduced into the Fc sequence, thereby making the mutated Fc more susceptible to the formation of homodimers or heterodimers. For example, Ridgway, Presta et al. 1996 and Carter 2001 mentioned the knob-hole model that utilizes the steric effect of amino acid side chain groups on the Fc contact interface, which makes it easier to form heterodimers between different Fc mutations; for example, in CN 1025583 55A or CN 103388013A, the ionic interaction force between the Fc contact interfaces is changed by changing the charges of the amino acids of the Fc contact interfaces, so that heterodimers (CN 102558355A) are more easily formed between different Fc mutation pairs, or homodimers (CN 103388013A) are more easily formed between Fc with the same mutation.

The immunoglobulin Fc region is preferably a human immunoglobulin Fc region, for example, an Fc region of human IgG1, IgG2, IgG3 or IgG4. In some specific embodiments, an amino acid sequence of the immunoglobulin Fc region is set forth in SEQ ID NO: 336.

In some specific embodiments, in the FXI binding protein of the present invention, the immunoglobulin Fc region (e.g., the Fc region of human IgG1) is linked directly or indirectly to the C-terminus of the immunoglobulin single variable domain (e.g., a VHH) via a linker (e.g., a peptide linker).

In some embodiments, the FXI binding protein of the present invention comprises an immunoglobulin single variable domain that specifically binds to FXI, which is linked directly or via a linker, to an immunoglobulin Fc region that allows the FXI binding protein to form a dimeric molecule comprising two FXI binding domains. Such a FXI binding protein is also referred to as a bivalent FXI binding protein. In some embodiments, the dimer is a homodimer. In some embodiments, the FXI binding protein of the present invention comprises two immunoglobulin single variable domains that specifically bind to FXI and an immunoglobulin Fc region that allows the FXI binding protein to form a dimeric molecule comprising four FXI binding domains, the two immunoglobulin single variable domains and the immunoglobulin Fc region being linked to each other directly or via a linker. Such a FXI binding protein is also referred to as a tetravalent FXI binding protein. In some embodiments, the dimer is a homodimer. In some embodiments, two immunoglobulin single variable domains in the FXI binding protein that specifically bind to FXI bind to different regions or different epitopes of FXI, respectively.

In some embodiments, the FXI binding protein of the present invention can inhibit the activity of FXI. In some embodiments, the FXI binding protein of the present invention can inhibit the coagulation function of FXI.

### Nucleic acid, vector and host cell

In another aspect, the present invention relates to a nucleic acid molecule encoding the FXI binding protein of the present invention. The nucleic acid of the present invention may be RNA, DNA or cDNA. According to an embodiment of the present invention, the nucleic acid of the present invention is a substantially-isolated nucleic acid.

The nucleic acid of the present invention may also be in the form of a vector, may be present in a vector and/or may be part of a vector, such as a plasmid, cosmid or YAC. The vector may especially be an expression vector, i.e., a vector that may provide the expression of the FXI binding protein *in vitro* and/or *in vivo* (i.e., in a suitable host cell, host organism and/or expression system). The expression vector typically comprises at least one nucleic acid of the present invention operably linked to one or more suitable expression control elements (e.g., promoters, enhancers and terminators). The selection of the elements and sequences thereof for expression in a particular host is common knowledge of those skilled in the art. Specific examples of control elements and other elements useful or necessary for expression of the FXI binding protein of the present invention include, for example, promoters, enhancers, terminators, integration factors, selection markers, leader sequences and reporter genes.

The nucleic acid of the present invention may be prepared or obtained by known means (e.g., by automated DNA synthesis and/or recombinant DNA techniques) based on information about the amino acid sequence of the polypeptide of the present invention given herein, and/or may be isolated from a suitable natural source.

In another aspect, the present invention relates to a recombinant host cell expressing or capable of expressing one or more FXI binding proteins of the present invention and/or comprising a nucleic acid or vector of the present invention. The host cells of the present invention are preferably bacterial cells, fungal cells or mammalian cells.

Suitable bacterial cells include cells of Gram-negative bacterial strains (e.g., Escherichia coli strains, Proteus strains and Pseudomonas strains) and Gram-positive bacterial strains (e.g., Bacillus strains, Streptomyces strains, Staphylococcus strains and Lactococcus strains). Suitable fungal cells include cells of species of Trichoderma, Neurospora and Aspergillus; or include cells of species of Saccharomyces (e.g., Saccharomyces cerevisiae), Schizosaccharomyces (e.g., Schizosaccharomyces pombe), Pichia (e.g., Pichia pastoris and Pichia methanolica) and Hansenula.

Suitable mammalian cells include, for example, HEK293 cells, CHO cells, BHK cells, HeLa cells, COS cells, and the like.

However, amphibian cells, insect cells, plant cells, and any other cells used in the art for expression of heterologous proteins may also be used in the present invention.

The present invention also provides a method for producing an FXI binding protein of the present invention, which generally comprises the following steps:
- culturing a host cell of the present invention under a condition that the FXI binding protein of the present invention is allowed to be expressed; and
- recovering the FXI binding protein expressed by the host cell from the culture; and
- optionally further purifying and/or modifying the FXI binding protein of the present invention. The FXI binding protein of the present invention may be produced intracellularly (e.g., in the cytoplasm, in the periplasm, or in inclusion bodies) in cells as described above, followed by isolation from the host cell and optionally further purification; or it may be produced extracellularly (e.g. in the medium in which the host cell is cultured), followed by isolation from the medium and optionally further purification.

Methods and reagents for recombinant production of polypeptides, e.g., specifically suitable expression vectors, transformation or transfection methods, selection markers, methods of inducing protein expression, culture conditions, etc., are known in the art. Similarly, protein isolation and purification techniques suitable for use in the methods for producing the FXI binding protein of the present invention are well known to those skilled in the art.

However, the FXI binding protein of the present invention may also be obtained by other protein production methods known in the art, such as chemical synthesis, including solid-phase or liquid-phase synthesis.

### Pharmaceutical composition

In another aspect, the present invention provides a composition, e.g., a pharmaceutical composition, comprising one or a combination of FXI binding proteins of the present invention formulated together with a pharmaceutically acceptable carrier. Such compositions may comprise one or a combination (e.g., two or more different) of FXI binding proteins of the present invention. For example, the pharmaceutical composition of the present invention may comprise a combination of antibody molecules that bind to different epitopes on a target antigen (FXI).

As used herein, the "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, an active compound, i.e., an antibody molecule, may be encapsulated into a material to protect the compound from acids and other natural conditions that may inactivate the compound.

The pharmaceutical composition of the present invention may also comprise a pharmaceutically acceptable antioxidant. Examples of pharmaceutically acceptable antioxidants include: (1) water-soluble antioxidants, such as ascorbic acid, cysteine hydrochloride, sodium bisulfate, sodium metabisulfite and sodium sulfite; (2) oil-soluble antioxidants, such as ascorbyl palmitate, butylated hydroxyanisole (BHA), butylated hydroxytoluene (BHT), lecithin, propyl gallate and α-tocopherol; and (3) metal chelating agents, such as citric acid, ethylenediamine tetraacetic acid (EDTA), sorbitol, tartaric acid and phosphoric acid.

These compositions may also comprise, for example, preservatives, wetting agents, emulsifying agents and dispersing agents.

Prevention of the presence of microorganisms can be ensured by sterilization procedures or by the inclusion of various antibacterial and antifungal agents, such as p-hydroxylbenzoate, chlorobutanol, phenol and sorbic acid. In many cases, the composition preferably comprises isotonic agents, for example, sugars, and polyalcohols such as mannitol, sorbitol, or sodium oxide. Prolonged absorption of injectable drugs can be achieved by adding into the composition an absorption delaying agent such as monostearate salts and gelatin.

The pharmaceutically acceptable carrier includes sterile aqueous solutions or dispersions and powders for the extemporaneous preparation of sterile injectable solutions or dispersions. The use of such media and reagents for pharmaceutically active substances is well known in the art. Conventional media or reagents, except incompatible with the active compounds, may be in the pharmaceutical composition of the present invention. Supplementary active compounds may also be incorporated into the composition.

Therapeutic compositions generally must be sterile and stable under the conditions of preparation and storage. The composition may be formulated as solutions, microemulsions, liposomes or other ordered structures suitable for high drug concentrations. The carrier may be a solvent or dispersing agent comprising, for example, water, ethanol, polyol (for example, glycerol, propylene glycol, and liquid polyethylene glycol), and suitable mixtures thereof. Proper fluidity can be maintained, for example, by the use of a coating, such as lecithin, by maintaining the required particle size in the case of dispersions and by the use of surfactants. Sterile injectable solutions may be prepared by incorporating an active compound at a required amount into an appropriate solvent with one or a combination of ingredients enumerated above, as required, followed by sterile microfiltration. Generally, dispersing agents are prepared by incorporating an active compound into a sterile carrier which comprises a basic dispersion medium and the required other ingredients from those enumerated above. For sterile powders used for preparing sterile injectable solutions, the preferred preparation methods are vacuum drying and freeze-drying (lyophilization) that yield a powder of the active ingredient plus any additionally-required ingredient from a previously sterile-filtered solution thereof.

The amount of active ingredient that can be combined with a carrier material to prepare a single dosage form will vary depending upon the treated subject and the particular mode of administration. The amount of active ingredient that can be combined with a carrier material to prepare a single dosage form will generally be an amount of the composition that produces a therapeutic effect. Typically, this amount ranges from about 0.01% to about 99%, for example from about 0.1% to about 70%, or from about 1% to about 30%, of the active ingredient, based on 100%, in combination with a pharmaceutically acceptable carrier.

Dosage regimens can be adjusted to provide the best desired response (e.g., therapeutic response). For example, a single bolus may be administered, several divided doses may be administered over time, or the dosage may be proportionally reduced or increased as required by the exigencies of the therapeutic situation. It is particularly advantageous to formulate a parenteral composition into a dosage unit form for ease of administration and uniformity of dosage. The dosage unit form as used herein refers to a physically discrete unit suitable as a unit dosage for the treated subject; each unit contains a predetermined quantity of active compound calculated to produce a required therapeutic effect in combination with a required pharmaceutical carrier. The specific description of the dosage unit form of the present invention is defined and directly dependent upon (a) the unique characteristics of the active compound and the particular therapeutic effect to be achieved, and (b) the limitations inherent in the art of formulating such active compounds for the treatment of sensitivity in individuals.

For administration of the antibody molecules, the dosage range is about 0.0001 mg/kg to 100 mg/kg, more usually 0.01 mg/kg to 30 mg/kg of the subject's body weight. For example, the dose may be 0.3 mg/kg of body weight, 1 mg/kg of body weight, 3 mg/kg of body weight, 5 mg/kg of body weight, 10 mg/kg, 20 mg/kg of body weight or 30 mg/kg of body weight or within a range of 1-30 mg/kg of body weight. Exemplary treatment regimens require administration once every week, once every two weeks, once every three weeks, once every four weeks, once every month, once every 3 months, once every 3-6 months, or require a slightly shorter administration interval (e.g., once every week to once every three weeks) followed by longer post-administration intervals (e.g., once every month to once every 3-6 months).

Or, the antibody molecules may be administered as a sustained-release preparation, in which case less frequent administration is required. The dose and frequency will vary depending on the half-life of the antibody molecules in the patient. Typically, human antibodies exhibit the longest half-life, followed by humanized antibodies, chimeric antibodies and non-human antibodies. The dosage and frequency of administration will vary depending on whether the treatment is prophylactic or therapeutic. In prophylactic use, relatively-low doses are administered at less frequent intervals over a longer period. Some patients continue to receive treatment for the remainder of their lives. In therapeutic use, it is sometimes desirable to administer higher doses at shorter intervals until progression of the disease is reduced or halted, preferably until the patient exhibits partial or complete improvement in disease symptoms. Thereafter, the administration to the patient may be carried out in a prophylactic regime. Actual dosage levels of active ingredients in the pharmaceutical composition of the present invention may be varied so as to obtain amounts of the active ingredients that are effective to achieve the required therapeutic response for a particular patient, composition and mode of administration, without being toxic to the patient. The selected dosage level will depend upon a variety of pharmacokinetic factors including the activity of the particular composition of the present invention employed, or an ester, salt or amide thereof, the route of administration, the time of administration, the rate of excretion of the particular compound employed, the duration of treatment, other drugs, compounds and/or materials used in combination with the particular composition employed, the age, sex, body weight, condition, general health and medical history of the patient being treated, and like factors well known in the medical arts.

The composition of the present invention may be administered by one or more routes of administration using one or more methods well known in the art. It should be appreciated by those skilled in the art that the route and/or mode of administration will vary depending on the desired result. Preferred routes of administration of the FXI binding protein of the present invention include intravenous, intramuscular, intradermal, intraperitoneal, subcutaneous, spinal, or other parenteral routes of administration, such as injection or infusion. The phrase "parenteral administration" as used herein refers to a mode of administration other than enteral and topical administrations, typically injection, including, but not limited to, intravenous, intramuscular, intraarterial, intramembranous, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Or, the FXI binding protein of the present invention may also be administered through non-parenteral routes, such as topical, epidermal or mucosal routes, e.g., intranasal, oral, vaginal, rectal, sublingual or topical administration.

### Treatment or prevention of disease

In one aspect, the present invention also provides a method for treating and/or preventing a thromboembolic condition or disease in a subject, comprising administering to the subject a therapeutically effective amount of the FXI binding protein of the present invention or the pharmaceutical composition of the present invention.

In some embodiments, the subject has or is at risk of myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, thromboembolism formed in extracorporeal circulation (such as cardiopulmonary bypass, hemodialysis and ECMO), arterial thrombosis, advanced renal disease, antiphospholipid syndrome, cerebralvascular accident, metastatic cancer or infectious disease.

In some embodiments, the subject has a pathological activation of FXI.

In one aspect, the present invention further provides use of the FXI binding protein of the present invention or the pharmaceutical composition of the present invention in the manufacture of a medicament for the treatment and/or prevention of a thromboembolic condition or disease. In some specific embodiments, the thromboembolic condition or disease is myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, thromboembolism formed in extracorporeal circulation (such as cardiopulmonary bypass, hemodialysis and ECMO), arterial thrombosis, advanced renal disease, antiphospholipid syndrome, cerebralvascular accident, metastatic cancer or infectious disease.

In one aspect, the present invention provides a method for inhibiting the activation of FXI (e.g., by factor XIIa (FXIIa)) in a subject, comprising: (a) selecting a subject in need of treatment, wherein the subject in need of treatment has or is at risk of thrombosis; and (b) administering to the subject an effective amount of the FXI binding protein of the present invention or the pharmaceutical composition of the present invention, thereby inhibiting the activation of FXI. In some embodiments, the subject in need of treatment is a subject having or at risk of: myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, thromboembolism formed in extracorporeal circulation (such as cardiopulmonary bypass, hemodialysis and ECMO), arterial thrombosis, advanced renal disease, antiphospholipid syndrome, cerebralvascular accident, metastatic cancer or infectious disease.

In some embodiments, the subject in need of treatment is a subject having a pathological activation of FXI.

In some embodiments, an effective amount of the FXI binding protein of the present invention or the pharmaceutical composition of the present invention is an amount sufficient to inhibit the activation of FXI by at least 10%, 20%, 30%, 40% or 50%.

In another aspect, the present invention provides a method for inhibiting coagulation and related thrombosis in a subject in need thereof without compromising hemostasis, which comprises administering to the subject a therapeutically effective amount of the FXI binding protein of the present invention or the pharmaceutical composition of the present invention, thereby inhibiting coagulation and related thrombosis in the subject without compromising hemostasis.

In some embodiments, the subject has or is at risk of: myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, thromboembolism formed in extracorporeal circulation (such as cardiopulmonary bypass, hemodialysis and ECMO), arterial thrombosis, advanced renal disease, antiphospholipid syndrome, cerebralvascular accident, metastatic cancer or infectious disease.

In some embodiments, the subject is a subject having a pathological activation of FXI.

In another aspect, the present invention also provides use of the FXI binding protein of the present invention or the pharmaceutical composition of the present invention in the manufacture of a medicament for the inhibition of coagulation and related thrombosis without compromising hemostasis.

In some embodiments of various aspects of the present invention, the FXI binding protein of the present invention or the pharmaceutical composition of the present invention is administered to the subject through parenteral administration.

### Detection

In another aspect, the present invention also provides a method for detecting the presence and/or an amount of FXI in a biological sample, which comprises contacting the biological sample and a control sample with the FXI binding protein of the present invention under a condition that a complex can be formed between the FXI binding protein of the present invention and FXI; and detecting the formation of a complex, wherein a difference in complex formation between the biological sample and the control sample is indicative of the presence and/or the amount of FXI in the sample.

In some embodiments, the FXI binding protein of the present invention is also conjugated with fluorescent dyes, chemical substances, polypeptides, enzymes, isotopes, labels and the like that can be used for detection or can be detected by other reagents.

### Kit

Also included within the scope of the present invention is a kit for use in the methods of the present invention, wherein the kit comprises the FXI binding protein of the present invention, and instructions for use. The kit typically comprises labels indicative of the intended use of kit contents. The term labels include any written or recorded materials provided on or with the kit or otherwise provided with the kit.

### Example

The present invention is further illustrated by the following examples; however, these examples should not be construed as limiting the present invention.

### Example 1: Screening of heavy chain single-domain antibodies against FXI

### 1.1. Library construction

5 mL bactrian camel arterial blood was collected in a vacuum blood collection tube before immunization, and the supernatant was collected as pre-immunization serum. For the primary immunization, a healthy 2-year-old Xinjiang bactrian camel was selected, 300 µg of recombinant human factor XI (hFXI, self-prepared, with sequence reference to Uniprot database, accession number P03951) was taken as an antigen and then uniformly mixed with a complete Freund's adjuvant in an equal volume, and after the protein was completely emulsified, multi-point injection was performed on the neck muscle of the bactrian camel; for the later-stage immunization, a same volume of antigen and incomplete Freund's adjuvant were uniformly mixed each time; and the immunization was performed once a week, and the bactrian camel was immunized six times in the later period. When the last immunization was completed, 5 mL bactrian camel arterial blood was collected in a vacuum blood collection tube, and the supernatant was collected as post-immunization serum.

Lymphocytes were separated by density gradient centrifugation, and total RNA was extracted using an RNA extraction kit provided by QIAGEN corporation. The extracted RNA was all reversely transcribed into cDNA using the Super-Script III FIRST STRANDSUPERMIX kit according to the instructions, and the nucleic acid fragment encoding the variable region of the heavy chain antibody was amplified by nested PCR.

The nucleic acid fragment of the heavy chain single-domain antibody of interest was recovered and then cloned into a vector pMECS used for phage display using restriction endonucleases PstI and NotI (purchased from NEB). The product was then electrotransformed into *E.coli* electroporation competent cells TG1, and an immune single-domain antibody phage display library against recombinant human factor XI was constructed and assayed. The size of the reservoir was calculated to be 1.5 × 10⁸ by gradient dilution plating. To check the insertion rate of the library, 50 clones were randomly selected for sequencing test, wherein 50 clones with correct foreign fragment insertions were found with a correct rate of 100%. By analyzing and comparing the DNA and amino acid sequences of the sequencing clones respectively, all the sequences were verified to be camel VHH sequences, and the diversity of the camel VHH sequences could be estimated to be more than 95%.

### 1.2. Panning of heavy chain single-domain antibody against FXI

For the first screening, a plate was coated with protein hApple-chis (self-prepared, sequence reference to Uniprot database, accession number P03951, in which the sequence of the first 387 amino acids was selected and then His-tag was added at the C-terminus of the sequence for purification) at 5 µg/well and left overnight at 4 °C. The next day after 2 hours blocking with 2% skim milk at room temperature, the plate was added with 100 µL phages (about 10⁸-10⁹pfu, from the hFXI-Chis single-domain antibody display library from 1.1.) and reacted at room temperature for 2 hours. Non-binding phages were washed off 25 times with PBST (0.05% Tween 20 in PBS). Finally, the phages specifically binding to hApple-chis were dissociated with Glycine (100 mM, pH 2.0).

For second screening, the plate was coated with protein mApple-chis (self-prepared, sequence reference to Uniprot database, accession number Q91Y47, in which the sequence of the first 389 amino acids was selected and then His-tag was added at the C-terminus of the sequence for purification) at 3 µg/well and left overnight at 4 °C. The next day after 2 hours blocking with 2% non-fat dry milk at room temperature, the plate was added with 100 µL phages (about 10⁸-10⁹pfu, from the hFXI-Chis single-domain antibody display library from 1.1.) and reacted at room temperature for 2 hours. Non-binding phages were washed off 25 times with PBST (0.05% Tween 20 in PBS). Finally, the phages specifically binding to mApple-chis were dissociated with Glycine (100 mM, pH 2.0) and infected with *E.coli* TG1 in logarithmic growth, and phages were generated and purified for the next round of screening. For the second round of screening, the plate was coated with protein hApple-chis at 10 µg/well followed by performing the above procedures.

Therefore, positive clones were enriched, and the purpose of screening FXI specific antibodies in an antibody library by using a phage display technology was fulfilled.

### 1.3. Screening of specific single positive clones using enzyme-linked immunosorbent assay (ELISA)

The FXI-binding positive phages obtained after panning were infected with blank *E. coli* and plated. Then 190 single colonies were randomly selected, named iFE 1 to iFE 190, and separately inoculated into 2TY-AG, and when the colonies were cultured to OD600 of about 0.8, IPTG was added to a final concentration of about 1 mM; after the expression was induced overnight at 25 °C, single-domain antibodies were expressed in *E.coli* periplasm; the next day, the bacteria were harvested and lysed, and the supernatant was collected and used for ELISA assay. The plates were coated with hFXI, hApple and mApple, respectively, and then reacted overnight at 4 °C, and the obtained sample lysis supernatant (the control group was *E.coli* lysis supernatant as a blank control) was added and reacted at room temperature for 2 hours. After washing, secondary antibody Goat anti-HA tag HRP (purchased from abeam) was added and reacted at room temperature for 2 hours. After washing, TMB chromogenic solution was added, absorbance values at 450 nm and 650 nm wavelengths were read, and a final absorbance value was obtained by subtracting the absorbance value at 650 nm wavelength from the absorbance value at 450 nm wavelength. When the OD value of the sample wells was more than 2 times larger than that of the control wells, the sample wells were determined as positive cloning wells. Positive clones were sent to Genewiz for sequencing.

The protein sequence of each clone was analyzed according to the sequence alignment software BioEdit. Clones with > 90% sequence homology of CDR1, CDR2 and CDR3 were considered to be identical antibody strains. A total of 23 strains of different antibodies were finally obtained. The binding assay results are shown in Table 1 below, and it can be seen that iFE96, iFE97, iFE148, iFE163, iFE166 and iFE168 bind to hFXI, hApple and mApple simultaneously, and iFE29, iFE30, iFE5, iFE7, iFE13, iFE15, iFE35, iFE56, iFE11, iFE17, iFE22, iFE43, iFE49, iFE50, iFE70, iFE108 and iFE128 bind to hFXI and hApple, without binding to mApple.

**Table 1. Binding properties of the 23 strains of antibodies**

| Sample | OD | | |
|---|---|---|---|
| | hFXI | hApple | mApple |
| iFE5 | 1.334 | 1.623 | 0.069 |
| iFE7 | 1.41 | 1.764 | 0.054 |
| iFE11 | 1.21 | 1.53 | 0.044 |
| iFE13 | 1.935 | 1.977 | 0.056 |
| iFE15 | 1.854 | 1.902 | 0.047 |
| iFE17 | 1.174 | 1.674 | 0.048 |
| iFE22 | 0.978 | 1.327 | 0.042 |
| iFE29 | 1.43 | 1.776 | 0.043 |
| iFE30 | 1.5 | 1.799 | 0.04 |
| iFE35 | 1.802 | 1.614 | 0.043 |
| iFE43 | 1.058 | 1.378 | 0.04 |
| iFE49 | 0.998 | 1.256 | 0.061 |
| iFE50 | 0.439 | 1.051 | 0.042 |
| iFE56 | 1.424 | 1.462 | 0.04 |
| iFE70 | 0.918 | 1.283 | 0.044 |
| iFE96 | 0.407 | 0.58 | 0.334 |
| iFE97 | 1.302 | 0.994 | 0.774 |
| iFE107 | 1.29 | 1.647 | 0.061 |
| iFE128 | 1.029 | 1.454 | 0.048 |
| iFE148 | 0.714 | 0.998 | 0.681 |
| iFE163 | 1.462 | 1.094 | 1.067 |
| iFE166 | 1.242 | 1.693 | 1.835 |
| iFE168 | 0.619 | 0.974 | 0.843 |

### 1.4. Prokaryotic expression and purification of positive clones

The single colonies of the positive clones (*E.coli* TG1 expression system, vector pMECS, with HA and HIS tags) obtained by screening in 1.3. were cultured overnight in 2TY-AG, and the seed solution was transferred to a 50-mL 2TY-AG medium; after the medium was cultured at 37 °C to OD600 of about 0.8, IPTG was added to a final concentration of about 1 mM, and expression was induced overnight at 25 °C. The next day, the bacteria were harvested, resuspended with a Tris buffer, and crushed through ultrasounds, and the obtained supernatant was purified by using His tag on the single-domain antibody and Ni-column affinity chromatography to obtain corresponding target protein.

### 1.5. Affinity assay of prokaryotic expression protein for hApple and mApple

Plates were coated with hApple and mApple proteins, respectively, at 0.5 µg/well and reacted overnight at 4 °C. The plates were added with a gradient dilution series of the candidate single-domain antibodies with His and HA tags obtained in 1.4. and reacted at room temperature for 2 hours. After washing, a chicken anti-HA-tag secondary antibody (streptavidin-HRP, abcam) labeled with horseradish peroxidase was added and reacted at room temperature for 2 hours. After washing, a chromogenic solution was added, absorbance values at 450 nm and 650 nm wavelengths were read, and a final absorbance value was obtained by subtracting the absorbance value at 650 nm wavelength from the absorbance value at 450 nm wavelength. Data processing and mapping analysis were carried out by using software SotfMax Pro v5.4, and through four-parameter fitting, binding curves of the candidate single-domain antibodies against FXI to hApple and mApple and EC₅₀ values were obtained so as to reflect the affinities of the candidate antibodies for hApple and mApple.

The results are shown in Table 2, and it can be seen that the candidate single-domain antibodies all bind to hApple, wherein iFE43, iFE50 and iFE96 have weak affinities for protein hApple; while iFE96, iFE97, iFE148, iFE163, iFE166 and iFE168 bind to mApple simultaneously.

### Table 2. Binding affinities of candidate antibodies for hApple and mApple

| Sample | EC₅₀ (ng/mL): with hApple | EC₅₀ (ng/mL): with mApple |
|---|---|---|
| iFE5 | 32.9 | - |
| iFE7 | 18.7 | - |
| iFE11 | 43.6 | - |
| iFE13 | 19.5 | - |
| iFE15 | 19.7 | - |
| iFE17 | 31.4 | - |
| iFE22 | 116.0 | - |
| iFE29 | 23.8 | - |
| iFE30 | 24.0 | - |
| iFE35 | 24.1 | - |
| iFE43 | Very weak binding with a maximum OD < 0.5 | - |
| iFE49 | 1160 | - |
| iFE50 | Very weak binding with a maximum OD < 0.5 | - |
| iFE56 | 323.0 | - |
| iFE70 | 60.3 | - |
| iFE96 | 1250 (OD 1.3 at 20 µg/mL) | OD 0.8 at 20 µg/mL |
| iFE97 | 735.0 | 1230 (OD 1.4 at 20 µg/mL) |
| iFE107 | 43.3 | - |
| iFE128 | 54.7 | - |
| iFE148 | 263 | 255 |
| iFE163 | 16.2 | 12.9 |
| iFE166 | 49.8 | 46.8 |
| iFE168 | 20.5 | 58.4 |

### Example 2: Preparation of FXI Single-Domain Antibody-Fc Fusion Protein Using Mammalian Cells

### 2.1. Preparation of Fc fusion plasmid of FXI single-domain antibody

Primers were designed to PCR amplify the FXI single-domain antibody VHH fragments (amino acid sequences set forth in SEQ ID NOs: 1-23, wherein individual amino acids on the FR2 region of partial sequences were mutated in order to improve protein stability), the fragments were fused with a DNA fragment encoding human IgG1-Fc (amino acid sequence set forth in SEQ ID NO: 336), and then cloned into a conventional mammalian expression vector to obtain a recombinant plasmid for expressing FXI single-domain antibody-Fc fusion protein in mammals. Wherein different VHH fragments were amplified using universal primers and fused with a DNA fragment of human IgG1-Fc. The universal primers were as follows:
Upstream primer cccACCGGTCAGGTGCAGCTGCAGGAGTC
Downstream primer cccGGATCCTGAGGAGACGGTGACCTGG

### 2.2. Preparation of FXI single-domain antibody-Fc fusion protein

The vector obtained by construction in 2.1. was transfected into HEK293 cells for transient expression of the antibody. The recombinant expression plasmids were diluted with Freestyle293 media followed by addition of PEI (polyethylenimine) solution required for transformation, and each group of plasmid/PEI mixture was added separately to HEK293 cell suspensions, placed at 37 °C, 5% CO₂, and cultured in suspension. After culturing for 5-6 days, the transient expression culture supernatant was collected and purified by Protein A affinity chromatography to obtain the target FXI single-domain antibody-Fc fusion Protein. The obtained protein purity was detected initially using SDS-PAGE and SEC-HPLC. The expression and purity of each protein were analyzed and shown in Table 3 below.

**Table 3. Results of one-step purification after transient transformation of the obtained anti-FXI single-domain antibody-Fc fusion protein**

| Antibody | Expression level (mg/L) | SDS-PAGE purity% | SEC purity (%) |
|---|---|---|---|
| iFE5-Fc | 413 | >95% | 99.1 |
| iFE7m-Fc | 3.8 | - | - |
| iFE13-Fc | 367 | >95% | 99.4 |
| iFE15m-Fc | 16 | - | - |
| iFE17EREG-Fc | 42.9 | >95% | - |
| iFE29m-Fc | 25 | - | - |
| iFE30m-Fc | 50 | - | - |
| iFE35-Fc | 397 | >95% | 98.4 |
| iFE49-Fc | 495 | >95% | 99.3 |
| iFE56-Fc | 412 | >95% | 97.5 |
| iFE96-Fc | 412 | >95% | 97.9 |
| iFE97-Fc | 378 | >95% | 99 |
| iFE148-Fc | 418 | >95% | 97.4 |
| iFE163-Fc | 292 | >95% | 93.2 |
| iFE166-Fc | 486 | >95% | 98.3 |
| iFE168-Fc | 368 | >95% | 98.3 |

It can be seen that the expression levels of the FXI single-domain antibody-Fc fusion proteins iFE7m-Fc, iFE15m-Fc, iFE17EREG-Fc, iFE29m-Fc and iFE30m-Fc were very low, the other expression levels were above 290 mg/L, and the target protein with stable concentration and high purity was obtained after one-step purification using Protein A affinity chromatography column.

### Example 3: Identification of Functions of FXI Single-Domain Antibody-Fc Fusion Protein

### 3.1. Binding curves of FXI single-domain antibody-Fc fusion protein to mApple and hApple

Plates were coated with proteins mApple and hApple, respectively, at 0.5 µg/well, and a blank control was set simultaneously, and the plates were reacted at 4 °C overnight. A gradient dilution series of the FXI single-domain antibody-Fc fusion proteins obtained in Example 2.2 were added and reacted at room temperature for 2 hours. After washing, Goat anti-human IgG-HRP (purchased from SIGMA) was added and reacted at room temperature for 2 hours. After washing, a chromogenic solution was added, absorbance values at 450 nm and 650 nm wavelengths were read, and a final absorbance value was obtained by subtracting the absorbance value at 650 nm wavelength from the absorbance value at 450 nm wavelength. Data processing and mapping analysis were carried out by using software SotfMax Pro v5.4, and through four-parameter fitting, binding curves of the antibodies to mApple and hApple and EC₅₀ values were obtained so as to reflect the affinities of the antibodies for mApple and hApple.

The results are shown in Table 4, wherein the antibodies all bound well to the protein hApple, with the antibodies iFE96, iFE97, iFE148, iFE163, iFE166 and iFE168 also binding well to the protein mApple.

**Table 4. Binding of FXI single-domain antibody-Fc fusion protein to mApple and hApple**

| Sample | EC₅₀ (ng/mL): with hApple | EC₅₀ (ng/mL): with mApple |
|---|---|---|
| iFE5-Fc | 30.9 | - |
| iFE13-Fc | 30.4 | - |
| iFE15m-Fc | 42.2 | - |
| iFE17EREG-Fc | 3850 (not reach a plateau, with OD value of 1.85 at a high point of 30 µg/mL) | OD value of 0.785 at a high point of 30 µg/mL |
| iFE29m-Fc | 58 | - |
| iFE30m-Fc | 40.6 | - |
| iFE35-Fc | 31.9 | - |
| iFE49-Fc | 65.7 | OD value of 0.105 at a high point of 30 µg/mL, substantially no binding |
| iFE56-Fc | 36.6 | - |
| iFE96-Fc | 27.6 | 33.1 |
| iFE97-Fc | 36.1 | 44.4 |
| iFE148-Fc | 25.4 | 33.9 |
| iFE163-Fc | 46.3 | 55.4 |
| iFE166-Fc | 44.8 | 47.4 |
| iFE168-Fc | 25.8 | 31.5 |

### 3.2. Assay of affinity of FXI single-domain antibody-Fc fusion protein (bio-layer interferometry BLI)

The binding kinetics of the FXI single-domain antibody-Fc fusion protein obtained in the above example to the recombinant proteins hApple and mApple were measured by bio-layer interferometry (BLI) using a molecular interaction instrument. The FXI single-domain antibody-Fc fusion proteins iFE5-Fc, iFE13-Fc, iFE35-Fc, iFE56-Fc, iFE97-Fc and iFE5-Fc were diluted to a final concentration of 10 µg/mL and directly immobilized onto a protein A biosensor; for kinetic measurement, hApple was diluted with 0.02% PBST20 to 5 concentrations of 200 nM, 100 nM, 50 nM, 25 nM and 12.5 nM, and mApple was diluted to 5 concentrations of 50 nM, 25 nM, 12.5 nM, 6.25 nM and 3.13 nM, followed by injection for 150 seconds, dissociation for 900 seconds, and then regeneration with 10 mM glycine-HCl (pH 1.7) for 5 seconds. The association rate (kon) and dissociation rate (kdis) were calculated using a simple one-to-one Languir binding model (Octet K2 data analysis software version 9.0 (Data analysis 9.0)). The equilibrium dissociation constant (KD) was calculated as the ratio of kdis/kon.

The results are shown in Tables 5 and 6, wherein Table 5 shows that the FXI single-domain antibody-Fc fusion proteins comparably bound to hApple, and Table 6 shows that the antibodies iFE97-Fc and iFE148-Fc had better affinities for mApple.

The positive control 14E11 was prepared by transient expression of 293 cells according to the above method after gene synthesis with reference to the sequences in the Patent WO2010080623.

**Table 5. Affinity for hApple**

| Antibody | KD (M) | kon(1/Ms) | kdis(1/s) |
|---|---|---|---|
| Positive control 14E11 | 2.10E-09 | 9.55E+04 | 2.01E-04 |
| iFE5-Fc | 1.77E-09 | 6.41E+04 | 1.14E-04 |
| iFE13-Fc | 6.59E-09 | 9.86E+04 | 6.50E-04 |
| iFE35-Fc | 2.02E-08 | 3.95E+04 | 7.98E-04 |
| iFE56-Fc | 2.27E-08 | 3.33E+04 | 7.55E-04 |
| iFE97-Fc | 1.61E-09 | 1.50E+05 | 2.43E-04 |
| iFE148-Fc | 9.52E-09 | 1.09E+05 | 1.03E-03 |

**Table 6. Affinity for mApple**

| Antibody | KD (M) | kon(1/Ms) | kdis(1/s) |
|---|---|---|---|
| iFE97-Fc | <1.0E-12 | 5.17E+05 | <1.0E-07 |
| iFE148-Fc | 3.14E-11 | 3.60E+05 | 1.13E-05 |

### 3.3. Assay of different epitopes of FXI single-domain antibody-Fc fusion protein binding to Apple proteins (bio-layer interferometry BLI: epitope binding)

The happle-chis-biotin and the mapple-chis-biotin were respectively diluted to 10 µg/mL with 0.02% PBST20 using in-tandem method and then immobilized onto a SA biosensor for 100 seconds at a height of about 1 nm. The FXI single-domain antibody-Fc fusion protein was diluted to 200 nM with 0.02% PBST20, and divided into two groups, wherein the antibody binding time is 300 seconds, and the regeneration solution was 10 mM glycine-HCl (pH 1.7); the first antibody (saturating antibody) bound to the sensor until saturation, after which the second antibody (competing antibody) competed with the first antibody at the same concentration, and the percentage was then calculated. The percentage calculation formula was Ab2 with Ab1/Ab2 without Ab1.

The measurement results are shown in Tables 7, 8 and 9. From the above results, it can be seen from Table 7 that iFE13-Fc, iFE35-Fc and 14E11 competed, the epitopes of iFE13-Fc overlapped with those of 14E11, and the epitopes of iFE35-Fc partially overlapped with those of 14E11; iFE56-Fc and iFE35-Fc competed, and the epitopes of iFE56-Fc were partially hindered from those of 14E11; iFE5-Fc and iFE30-Fc competed, and their epitopes were identical and did not overlap with those of 14E11; it can be seen from Tables 8 and 9 that iFE97-Fc and iFE163-Fc competed, and their epitopes were identical and did not overlap with those of 14E11; iFE148-Fc recognized both human and mouse, and its epitopes did not overlap with those of other single-domain antigens and 14E11.

**Table 7. hApple**

| Percentage = Ab2 with Ab1/Ab2 without Ab1 | | | | | | |
|---|---|---|---|---|---|---|
| Ab1/Ab2 | 14E11 | iFE13-Fc | iFE30m-Fc | iFE56 -Fc | iFE5-Fc | iFE35-Fc |
| 14E11 | 5.42% | 12.09% | 92.11% | 64.30% | 83.85% | -14.47% |
| iFE13-Fc | 2.99% | 5.06% | 96.42% | 90.89% | 89.67% | 70.81% |
| iFE30m-Fc | 111.51% | 139.04% | 9.00% | 97.01% | 6.70% | 89.95% |
| iFE56-Fc | 117.47% | 143.68% | 101.70% | 7.12% | 91.94% | 4.95% |
| iFE5-Fc | 116.46% | 149.01% | 14.88% | 99.49% | 7.09% | 92.77% |
| iFE35-Fc | 25.16% | 126.05% | 102.20% | 15.33% | 91.29% | 8.49% |

**Table 8. hApple**

| Percentage = Ab2 with Ab1/Ab2 without Ab1 | | | | |
|---|---|---|---|---|
| Ab1/Ab2 | 14E11 | iFE97-Fc | iFE148-Fc | iFE163-Fc |
| 14E11 | 5.32% | 101.95% | 76.49% | 86.98% |
| iFE97-Fc | 105.13% | 6.97% | 88.05% | 10.20% |
| iFE148-Fc | 100.87% | 110.12% | 11.95% | 84.32% |
| iFE163-Fc | 105.98% | 7.32% | 87.43% | 9.35% |

**Table 9. mApple**

| Percentage = Ab2 with Ab1/Ab2 without Ab1 | | | |
|---|---|---|---|
| Ab1/Ab2 | IFE97-Ld-Fc | IFE148-Ld-Fc | IFE163-Ld-Fc |
| IFE97-Ld-Fc | 8.37% | 99.32% | 11.25% |
| IFE148-Ld-Fc | 113.04% | 17.38% | 77.35% |
| IFE163-Ld-Fc | 3.53% | 90.19% | 6.77% |

3.4. Assay of non-specific binding of FXI single-domain antibody-Fc fusion protein to null cells CHOK1 null cells and 293F null cells were resuspended in 3% BSA-PBS, the number of cells was adjusted to 6 × 10⁶ cell/mL, and the final concentration of FXI single-domain antibody-Fc fusion protein was selected to be 100 µg/mL according to the results of Example 3.1, while negative and blank controls were set and incubated in an ice bath for 60 minutes. After washing, Biolegend secondary antibody FITC anti-human IgG Fc was added in an ice bath for 30 minutes. After washing, the cells were resuspended in 300 µL of 1% PBS-BSA buffer and detected using a flow cytometer.

The results are shown in Tables 10 and 11, wherein none of the candidate antibodies specifically bound to the null cells.

**Table 10**

| Sample name | Concentration (µg/mL) | 293F | CHOK1 |
|---|---|---|---|
| blank | / | 5.82 | 4.62 |
| negative | / | 6.40 | 4.87 |
| iFE5-Fc | 100 | 7.02 | 5.06 |
| iFE13-Fc | 100 | 6.77 | 5.15 |
| iFE35-Fc | 100 | 6.67 | 5.20 |
| iFE56-Fc | 100 | 6.53 | 5.00 |
| iFE97-Fc | 100 | 7.11 | 5.78 |
| iFE148-Fc | 100 | 7.09 | 5.34 |
| iFE30m-Fc | 100 | 8.16 | 9.23 |
| iFE163-Fc | 100 | 7.56 | 7.04 |
| iFE49-Fc | 100 | 28.88 | 30.52 |

### 3.5. Identification of blocking activity of FXI single-domain antibody-Fc fusion protein against FXI (APTT assay)

The pre-warmed blood plasma and a reagent were quickly mixed and incubated by using an optical detection method, an absorbance value of the blood plasma was detected at a wavelength of 660 nm, the fibrinogen was converted into the fibrin over the incubation time to increase the turbidity of the mixture such that the intensity of scattered light could be changed, and the changed intensity of the scattered light due to the increase of the turbidity of the sample was determined by using an instrument so as to determine the coagulation time. The commercial coagulation factor XI was taken as a standard substance, and the activity of the sample was calculated from a standard curve drawn by a coagulation method using the standard curve with the coagulation time as the Y-axis and the activity percentage of a reference plasma as the X-axis.

The antibodies were each diluted to 1 µg/mL with FXI-deficient plasma, and meanwhile, a buffer was taken as a negative control; 50 µL of processed samples were separately taken and incubated at 37 °C for 2 hours, then the samples were loaded to a full-automatic coagulator and automatically mixed and incubated with Dade Actin Activated Cephaloplastin Reagent, Calcium Chloride Solution and FACTOR IX DEFICIENT reagent, and then the absorbance values were detected at the wavelength of 660 nm. The results are shown in Table 11 below, wherein iFE15, iFE29, iFE96, iFE166 and iFE168 had no blocking activity.

**Table 11**

| Sample name | Coagulation time (s) | Activity |
|---|---|---|
| Without addition of antibodies | 60.9 | 88.6% |
| iFE13-Fc | 69.9 | 48.1% |
| iFE15m-Fc | 61 | 88.0% |
| iFE29m-Fc | 62.2 | 80.7% |
| iFE30m-Fc | 67.2 | 57.2% |
| iFE56-Fc | 70.4 | 46.6% |
| iFE5-Fc | 64.9 | 66.8% |
| iFE35-Fc | 70 | 47.7% |
| iFE96-Fc | 62.5 | 79.0% |
| iFE97-Fc | 67.9 | 54.7% |
| iFE148-Fc | 64.2 | 70.1% |
| iFE163-Fc | 68.4 | 52.9% |
| iFE166-Fc | 61.9 | 82.5% |
| iFE168-Fc | 61.7 | 83.7% |

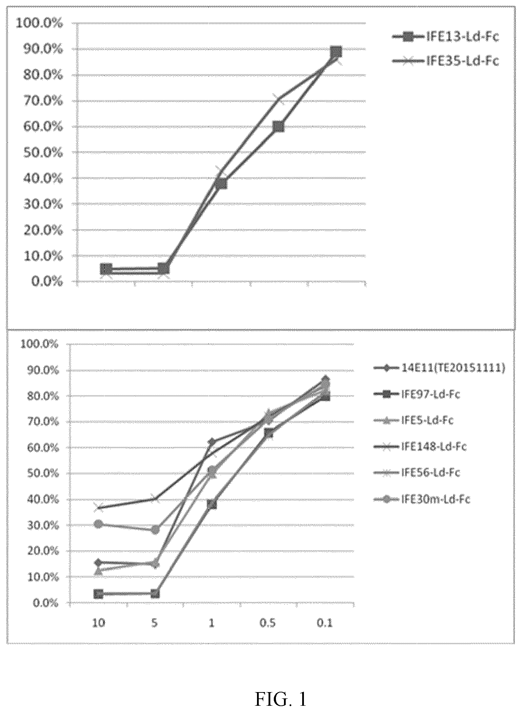

According to the results in Table 11, the antibodies iFE13, iFE35, iFE97, iFE5, iFE148, iFE56 and iFE30 were serially diluted with standard human plasma to detect activity curves, and meanwhile, a positive control 14E11 was set, and as shown in FIG. 1, it can be seen that the inhibitory effects of iFE5, iFE13, iFE35, iFE56 and iFE97 were significantly better than those of the positive control 14E11; the remaining antibodies iFE148 and iFE30 as well as the positive control exhibited comparable inhibitory effects.

### 3.6. Binding of FXI single-domain antibodies to different Apple domains

The FXI single-domain anti-Fc fusion protein obtained in the above example and the positive control 14E11 were selected and their binding kinetics to different human Apple domain proteins were examined using bio-layer interferometry (BLI). The sequences of different Apple domain proteins were from Uniprot (accession number P03951), with some Apple domains having His-tag added to their C-terminus for purification (with a Chis in the name) and with some Apple domains having mouse Fc fragments added to their C-terminus for purification (with a muFc in the name). The proteins were obtained by transient transfection in 293 cells and further purification by IMAC. The specific procedures were similar to those described above, and self-prepared hApple1-2muFc, hApple2-3muFc, hApple3-4muFc, hApple2-chis, hApple4-chis, hApple1-muFc and hApple3-muFc were taken as test substances.

The binding of the candidate FXI single-domain antibody-Fc fusion proteins to different Apple domains is shown in Tables 12 and 13 below.

**Table 12**

| | hApple1-2muFc | hApple2-3muFc | hApple3-4muFc | hApple1-muFc | hApple4-chis |
|---|---|---|---|---|---|
| 14E11 | Binding | Binding | No binding | No binding | No binding |
| iFE5-Fc | No binding | No binding | Binding | No binding | Binding |
| iFE13-Fc | Binding | Binding | No binding | No binding | No binding |
| iFE35-Fc | Binding | No binding | No binding | No binding | No binding |
| iFE56-Fc | Binding | Binding | No binding | No binding | No binding |
| iFE97-Fc | No binding | Binding | Binding | No binding | No binding |
| iFE148-Fc | No binding | Binding | No binding | No binding | No binding |

**Table 13**

| | hApple2-chis(KD, M) | hApple4-chis(KD, M) | hApple3-muFc(KD, M) |
|---|---|---|---|
| 14E11 | 2.15E-09 | No binding | No binding |
| iFE5-Fc | No binding | 4.29E-10 | No binding |
| iFE13-Fc | 7.71E-10 | No binding | No binding |
| iFE35-Fc | 8.20E-07 | No binding | No binding |
| iFE56-Fc | 3.02E-09 | No binding | No binding |
| iFE97-Fc | No binding | No binding | <1.0E-12 |
| iFE148-Fc | No binding | No binding | No binding |

### Example 4: Humanization of FXI Single-Domain antibodies

The humanization was achieved by humanization of amino acids on the protein surface (resurfacing) and the VHH humanized universal framework transplantation method (CDR grafting to a universal framework).

The humanization steps were as follows: antibody strains iFE5, iFE13, iFE35, iFE56, iFE97 and iFE148 were homologously modeled using modeling software Modeller 9. The reference homology sequence was NbBcII10 antibody (PDB numbered as 3DWT), and the relative solvent accessibility of amino acids was calculated from a three-dimensional structure of the protein. If an amino acid of the antibody strains iFE5, iFE13, iFE35, iFE56, iFE97 and iFE148 was exposed to a solvent, the amino acid was substituted with an amino acid at the same position as the reference human antibody 10HQ sequence, and finally, all substitutions were completed. The specific steps of the VHH humanized universal framework transplantation method were as follows: firstly, a universal humanized VHH framework hNbBcII10FGLA (PDB No. 3EAK) designed by Cécile Vincke et al. based on sequence homology was obtained, protein surface amino acid humanization was performed with reference to a humanized antibody 10HQ based on a nanobody NbBcII10 antibody (PDB No. 3DWT), and modification was performed on partial amino acid VLP of VHH sequence framework 1 (framework 1), partial amino acid GL of VHH sequence framework 2 (framework 2), partial amino acid RSKRAAV of VHH sequence framework 3 (framework 3), and amino acid L of VHH sequence framework 4 (framework 4). hNbBcII10FGLA was directly taken as a framework to replace the CDRs with the CDR regions of antibody strains iFE5, iFE13, iFE35, iFE56, iFE97 and iFE148, thereby completing humanization of the antibodies.

Antibody strains iFE5, iFE13, iFE35, iFE56, iFE97 and iFE148 were humanized to obtain humanized variants huFE of the six antibody strains, respectively. The sequences of these humanized variants are suitable for SEQ ID NOs: 300-335, respectively.

### Example 5: Preparation of Humanized Single-Domain Antibody-Fc Fusion Protein

### 5.1. Preparation of humanized single-domain antibody-Fc fusion plasmid

The huFE humanized sequences in Example 4 were commissioned to Suzhou Hongxun Biotechnology Co., Ltd. for gene synthesis, with enzyme digestion sites added at both ends.

The huFE single-domain antibody VHH fragment was subjected to double digestion, fused with a DNA fragment for encoding human IgG1FC, and cloned into a conventional mammal expression vector, so as to obtain a recombinant plasmid for expressing the huFE single-domain antibody-Fc fusion protein in a mammal.

### 5.2. Preparation of humanized single-domain antibody-Fc fusion protein

The vector obtained by construction in 4.1. was transfected into HEK293 cells for transient expression of the antibody. The recombinant expression plasmids were diluted with Freestyle293 media followed by addition of PEI (polyethylenimine) solution required for transformation, and each group of plasmid/PEI mixture was added separately to HEK293 cell suspensions, placed at 37 °C, 5% CO₂, and cultured at 130 rpm. Four hours later, the mixture was supplemented with EXCELL293 medium, added with 2 mM glutamine, and cultured at 130 rpm. After 24 hours, 3.8 mM VPAwas added, and after 72 hours, 4 g/L glucose was added. After culturing for 5-6 days, the transient expression culture supernatant was collected and purified by Protein A affinity chromatography to obtain the target huFE single-domain antibody-Fc fusion Protein. The protein was initially examined for purity by SDSPAGE and SECHPLC. The expression level of each protein exceeded 350 mg/L, and the SEC purity after one-step purification was more than 95%.

### Example 6: Identification of Functions of huFE Single-Domain Antibody-Fc Fusion Protein

### 6.1. Affinity of the huFE single-domain antibody-Fc fusion protein

The binding kinetics of the huFE single-domain antibody-Fc fusion protein obtained in the above example to hApple-CHis protein were measured by bio-layer interferometry (BLI) using a molecular interaction instrument. The huFE single-domain antibody-Fc fusion protein obtained in Example 5.2 was diluted to a final concentration of 10 µg/mL and directly immobilized onto an AHC biosensor, and for kinetic measurement, the hApple-CHis protein was diluted to 5 concentrations of 200 nM, 100 nM, 50 nM, 25 nM and 12.5 nM, respectively, with a 60-second baseline, binding for 120 seconds, and then dissociation for 900 seconds. The diluent was a kinetic buffer, the regeneration solution was glycine-HCl (pH 1.7), and the neutralization solution was the diluent. The biosensor was Protein A biosensor. The association rate (kon) and dissociation rate (kdis) were calculated using a simple one-to-one Languir binding model (Octet K2 data analysis software version 9.0 (Data analysis 9.0)). The equilibrium dissociation constant (KD) was calculated as the ratio of kdis/kon.

The measured affinity of the huFE single-domain antibody-Fc fusion protein for the hApple-Chis protein is shown in Table 14.

**Table 14**

| Sample | KD (M) | Sample | KD (M) |
|---|---|---|---|
| huFE35huv1-Ld-Fc | <1.0E-12 | huFE35huv2-Ld-Fc | 1.21E-12 |
| huFE13huv1-Ld-Fc | 7.64E-10 | huFE13huv2-Ld-Fc | 6.64E-10 |
| huFE56huv1-Ld-Fc | <1.0E-12 | huFE56huv2-Ld-Fc | <1.0E-12 |
| huFE97huv1-Ld-Fc | <1.0E-12 | huFE97huv2-Ld-Fc | <1.0E-12 |
| huFE148huv1-Ld-Fc | 3.70E-10 | huFE148huv2-Ld-Fc | 4.50E-10 |
| huFE5huv1-Ld-Fc | <1.0E-12 | huFE5huv2-Ld-Fc | 2.31E-12 |
| huFE35huv3-Ld-Fc | <1.0E-12 | huFE35huv4-Ld-Fc | 1.54E-12 |
| huFE13huv3-Ld-Fc | 9.30E-10 | huFE13huv4-Ld-Fc | 8.23E-10 |
| huFE56huv3-Ld-Fc | 2.10E-12 | huFE56huv4-Ld-Fc | 1.33E-12 |
| huFE97huv3-Ld-Fc | 1.42E-12 | huFE97huv4-Ld-Fc | <1.0E-12 |
| huFE148huv3-Ld-Fc | 3.68E-10 | huFE148huv4-Ld-Fc | 5.23E-10 |
| huFE5huv3-Ld-Fc | 1.44E-12 | huFE5huv4-Ld-Fc | 2.86E-12 |
| huFE35huv5-Ld-Fc | <1.0E-12 | huFE35huv6-Ld-Fc | 3.24E-12 |
| huFE13huv5-Ld-Fc | 8.56E-10 | huFE13huv6-Ld-Fc | 9.12E-10 |
| huFE56huv5-Ld-Fc | 3.21E-12 | huFE56huv6-Ld-Fc | 3.45E-12 |
| huFE97huv5-Ld-Fc | <1.0E-12 | huFE97huv6-Ld-Fc | 1.33E-12 |
| huFE148huv5-Ld-Fc | 4.23E-10 | huFE148huv6-Ld-Fc | 4.87E-10 |
| huFE5huv5-Ld-Fc | 1.56E-12 | huFE5huv6-Ld-Fc | <1.0E-12 |

The binding kinetics of the huFE single-domain antibody-Fc fusion protein obtained in the above example to hFXI-CHis protein were measured by bio-layer interferometry (BLI) using a molecular interaction instrument. The specific procedures were same as those described above, and the hFXI-Chis protein was taken as a test substance. 4E11 (as described above) and B1213790-F11a (self-prepared according to the sequence in WO2018134184) were taken as controls.

The measured affinity of the huFE single-domain antibody-Fc fusion protein for the hFXI-CHis protein is shown in Table 15.

**Table 15**

| Sample | KD (M) | Sample | KD (M) |
|---|---|---|---|
| huFE35huv1-Ld-Fc | 1.02E-08 | huFE35huv2-Ld-Fc | 2.55E-08 |
| huFE13huv1-Ld-Fc | 8.84E-09 | huFE13huv2-Ld-Fc | 1.05E-08 |
| huFE56huv1-Ld-Fc | 3.97E-09 | huFE56huv2-Ld-Fc | 4.21E-09 |
| huFE97huv1-Ld-Fc | 2.67E-09 | huFE97huv2-Ld-Fc | 2.51E-09 |
| huFE148huv1-Ld-Fc | 2.64E-09 | huFE148huv2-Ld-Fc | 4.56E-09 |
| huFE5huv1-Ld-Fc | 6.56E-10 | huFE5huv2-Ld-Fc | 6.12E-10 |
| huFE35huv3-Ld-Fc | 9.65E-09 | huFE35huv4-Ld-Fc | 3.13E-08 |
| huFE13huv3-Ld-Fc | 6.84E-09 | huFE13huv4-Ld-Fc | 8.84E-09 |
| huFE56huv3-Ld-Fc | 5.97E-09 | huFE56huv4-Ld-Fc | 3.97E-09 |
| huFE97huv3-Ld-Fc | 1.98E-09 | huFE97huv4-Ld-Fc | 2.67E-09 |
| huFE148huv3-Ld-Fc | 3.45E-09 | huFE148huv4-Ld-Fc | 2.64E-09 |
| huFE5huv3-Ld-Fc | 4.56E-10 | huFE5huv4-Ld-Fc | 6.56E-10 |
| huFE35huv5-Ld-Fc | 1.02E-08 | huFE35huv6-Ld-Fc | 1.02E-08 |
| huFE13huv5-Ld-Fc | 8.84E-09 | huFE13huv6-Ld-Fc | 8.84E-09 |
| huFE56huv5-Ld-Fc | 3.97E-09 | huFE56huv6-Ld-Fc | 3.97E-09 |
| huFE97huv5-Ld-Fc | 2.67E-09 | huFE97huv6-Ld-Fc | 2.67E-09 |
| huFE148huv5-Ld-Fc | 2.64E-09 | huFE148huv6-Ld-Fc | 2.64E-09 |
| huFE5huv5-Ld-Fc | 6.56E-10 | huFE5huv6-Ld-Fc | 6.56E-10 |
| 14E11 | 3.02E-09 | B1213790-F11a | 4.12E-09 |

The results show that the humanized single-domain antibody-Fc fusion protein could well bind to hApple-CHis and hFXI-CHis, and no significant difference existed between various humanized versions. In addition, they had comparable binding ability to the two control antibodies.

A part of the huFE single-domain anti-Fc fusion proteins obtained in the above example were selected, and the binding kinetics of the huFE single-domain anti-Fc fusion proteins to the FXI proteins of New Zealand rabbits were examined by bio-layer interferometry (BLI). The specific procedures were same as those described above, and the self-prepared FXI proteins of the New Zealand rabbits were taken as test substances (related protein sequence reference to Uniprot database, accession number Q95ME7).

The measured affinity of the huFE single-domain antibody-Fc fusion protein for the RabFXI-Apple-CHis protein is shown in Table 16 below.

**Table 16**

| Sample | KD (M) | Sample | KD (M) |
|---|---|---|---|
| huFE35huv1-Ld-Fc | / | huFE35huv2-Ld-Fc | / |
| huFE13huv1-Ld-Fc | 2.95E-08 | huFE13huv2-Ld-Fc | 3.15E-08 |
| huFE56huv1-Ld-Fc | / | huFE56huv2-Ld-Fc | / |
| huFE97huv1-Ld-Fc | 8.78E-10 | huFE97huv2-Ld-Fc | 9.60E-10 |
| huFE148huv1-Ld-Fc | 7.83E-09 | huFE148huv2-Ld-Fc | 7.21E-09 |
| huFE5huv1-Ld-Fc | 2.87E-07 | huFE5huv2-Ld-Fc | 4.51E-07 |

Apart of the huFE single-domain anti-Fc fusion proteins obtained in the above example were selected, and the binding kinetics of the huFE single-domain anti-Fc fusion proteins to the FXI proteins of cynomolgus monkeys were examined by bio-layer interferometry (BLI). The specific procedures were same as those described above, and the self-prepared FXI proteins of the cynomolgus monkeys were taken as test substances (related protein sequence reference to Uniprot database, accession number A0A2K5VVK2).

The measured affinity of the huFE single-domain antibody-Fc fusion protein for the cynomolgus monkey FXI-CHis protein is shown in Table 17.

**Table 17**

| Sample | KD (M) | Sample | KD (M) |
|---|---|---|---|
| huFE35huv1-Ld-Fc | <1.0E-12 | huFE35huv2-Ld-Fc | 1.24E-12 |
| huFE13huv1-Ld-Fc | 1.27E-08 | huFE13huv2-Ld-Fc | 2.06E-08 |
| huFE56huv1-Ld-Fc | 5.93E-09 | huFE56huv2-Ld-Fc | 7.23E-09 |
| huFE97huv1-Ld-Fc | <1.0E-12 | huFE97huv2-Ld-Fc | <1.0E-12 |
| huFE148huv1-Ld-Fc | 9.87E-10 | huFE148huv2-Ld-Fc | 8.25E-10 |
| huFE5huv1-Ld-Fc | / | huFE5huv2-Ld-Fc | / |

The above results indicated that most candidate antibodies could simultaneously recognize coagulation F11 factors of humans, New Zealand rabbits and cynomolgus monkeys. But the binding was relatively reduced for the New Zealand rabbits. In addition, antibodies 35 and 56 were substantially inactive in the New Zealand rabbits, while antibody 5 was substantially inactive in the cynomolgus monkeys. There was no significant difference in activity between the various humanized versions of the proteins.

A part of the huFE single-domain anti-Fc fusion proteins obtained in the above example were selected, and the binding kinetics thereof to activated human factor 11 protein (hFXIa) were examined by bio-layer interferometry (BLI). The specific procedures were same as those described above, and commercially available hFXIa was taken as a test substance. 14E11 (as described above) and B1213790-F11a (as described above) were taken as controls.

The measured affinity of the huFE single-domain antibody-Fc fusion protein for the hFXIa protein is shown in Table 18.

**Table 18**

| Sample | KD (M) |
|---|---|
| huFE35huv1-Ld-Fc | 4.55E-08 |
| huFE13huv1-Ld-Fc | 6.93E-09 |
| huFE56huv1-Ld-Fc | 2.88E-08 |
| huFE97huv1-Ld-Fc | 1.42E-09 |
| huFE148huv1-Ld-Fc | 5.05E-09 |
| 14E11 | 5.73E-10 |
| B1213790-F11a | 6.85E-11 |

It can be seen from the above results that the humanized FXI single-domain antibody-Fc fusion protein can efficiently bind to activated FXI factor. The binding ability thereof is lower than that of B1213790-F11a which directly binds to the FXI factor activation site.

At the same time, combined with previous binding results to unactivated FXI factor, 14E11, which also bounds to Apple domain (Apple2), had improved binding ability to the activated FXIa. However, the binding of the candidate antibodies of the present invention to activated FXIa was comparable to the binding activity thereof to non-activated FXI, or the binding thereof to activated FXIa was reduced. The results showed that, in another aspect, even though there was some crossover with 14E11 at the binding epitopes, the binding patterns of the candidate antibodies were significantly different.

### 6.2. Inhibitory effect of the huFE single-domain antibody-Fc fusion protein against human FXI factor activity

The activity of human FXI in standard human plasma (purchased from WHO) was 92%, and the effect of inhibiting the activity of FXI factors in the plasma after adding different single-domain antibody-Fc fusion proteins and a control (14E11) was detected by matching with FXI-deficient plasma. The results of the activity of FXI factors mixed with different antibodies are shown in Table 19 and FIG. 2. These humanized FXI antibodies all have better inhibitory activity results against FXI factors. The 14E11 protein was taken as a positive control.

**Table 19**

| Concentration (µg/mL) | 14E11 | huFE5huvl-Ld-Fc | huFE35huvl-Ld-Fc | huFE13huv1-Ld-Fc | huFE56huvl-Ld-Fc | huFE97huvl-Ld-Fc | huFE148huv1-Ld-Fc |
|---|---|---|---|---|---|---|---|
| 10 | 8.15% | 6.15% | 1.90% | 11.25% | 1.50% | 1.50% | 22.20% |
| 5 | 8.70% | 7.50% | 1.90% | 13.10% | 1.70% | 1.60% | 30.95% |
| 1 | 64.00% | 47.35% | 33.00% | 29.75% | 33.40% | 22.60% | 48.30% |
| 0.5 | 80.00% | 68.90% | 58.35% | 61.15% | 61.70% | 60.85% | 69.80% |
| 0.1 | 92.55% | 87.15% | 81.80% | 80.00% | 87.00% | 85.15% | 84.90% |

### Example 7: Preparation of huFE Bispecific Antibody-Fc Fusion Protein Using Mammalian Cells

### 7.1. Preparation of huFE bispecific antibody-Fc fusion plasmids

The gene of the huFE single-domain antibody-Fc fusion protein obtained in Example 4 was subjected to molecular cloning to obtain a recombinant plasmid for expressing the huFE bispecific antibody-Fc fusion protein in mammals, for use in the preparation of bispecific antibody proteins as follows: huFE97n13-Ld-Fc, huFE13n97-Ld-Fc, huFE97n56-Ld-Fc, huFE56n97-Ld-Fc, huFE97di-Ld-Fc, huFE56n13-Ld-Fc, huFE13n56-Ld-Fc, huFE97n148-Ld-Fc, huFE148n97-Ld-Fc, huFE5n97-Ld-Fc, huFE97n5-Ld-Fc, huFE148n5-Ld-Fc, huFE5n148-Ld-Fc, huFE148n56-Ld-Fc, huFE56n148-Ld-Fc, huFE56n5-Ld-Fc, huFE5n56-Ld-Fc, huFE5n13-Ld-Fc and huFE13n5-Ld-Fc.

Meanwhile, a tetravalent monospecific antibody of HuFE97di-Ld-Fc was also obtained by using the above method or taken as a control antibody.

### 7.2. Preparation of huFE bispecific antibody-Fc fusion protein

The vector obtained by construction in 7.1. was transfected into HEK293 cells for transient expression of the antibody. The recombinant expression plasmids were diluted with Freestyle293 media followed by addition of PEI (polyethylenimine) solution required for transformation, and each group of plasmid/PEI mixture was added separately to HEK293 cell suspensions, placed at 37 °C, 5% CO₂, and cultured in suspension. After culturing for 5-6 days, the transient expression culture supernatant was collected and purified by Protein A affinity chromatography to obtain the target huFE bispecific antibody-Fc fusion protein. The protein was initially examined for purity by SDSPAGE and SECHPLC.

The expression levels of all proteins were between 250 mg/L and 400 mg/L, and the SEC purity after one-step purification was more than 95%. The results preliminarily prove that the bispecific antibodies are excellent in solubility and stability, and are suitable for being taken as drug candidate molecules.

### Example 8: High Temperature Acceleration of huFE Bispecific Antibody-Fc Fusion Protein

The huFE bispecific antibody-Fc fusion proteins obtained in the above examples were each taken at 12 mg, concentrated to 10 mg/mL by ultrafiltration, and dissolved in PBS. Then, the mixture was placed at 40 °C, and was periodically sampled to detect variations in protein content, SEC purity and the like so as to examine the stability of the proteins. The results are shown in Tables 20 and 21 below.

**Table 20. Detection of protein contents of 7 proteins at OD 280 nm after acceleration**

| | Protein | Day 0 | Day 20 | Content (%) |
|---|---|---|---|---|
| | | Protein content (mg/mL) | Protein content (mg/mL) | |
| 1 | huEF97n13-Ld-Fc | 10.131 | 10.432 | 103.0 |
| 2 | huEF13n97-Ld-Fc | 10.126 | 10.282 | 101.5 |
| 3 | huEF97n56-Ld-Fc | 10.097 | 10.475 | 103.7 |
| 4 | huEF56n97-Ld-Fc | 10.160 | 10.446 | 102.8 |
| 5 | HuFE97di-Ld-Fc | 9.967 | 10.856 | 108.9 |
| 6 | huEF56n13-Ld-Fc | 10.158 | 10.266 | 101.1 |
| 7 | huEF13n56-Ld-Fc | 10.088 | 10.245 | 101.5 |
| 8 | huEF56n13-Ld-Fc | 10.234 | 10.356 | 101.2 |
| 9 | huEF13n56-Ld-Fc | 10.212 | 10.412 | 102.0 |

**Table 21. SE-HPLC assay**

| | Protein | 0D | 20D |
|---|---|---|---|
| | | Main peak (%) | Main peak (%) |
| 1 | huEF97n13-Ld-Fc | 94.62 | 88.61 |
| 2 | huEF13n97-Ld-Fc | 97.54 | 96.42 |
| 3 | huEF97n56-Ld-Fc | 92.03 | 87.5 |
| 4 | huEF56n97-Ld-Fc | 95.61 | 90.95 |
| 5 | huFE97di-Ld-Fc | 93.87 | 90.56 |
| 6 | huEF56n13-Ld-Fc | 95.19 | 94.19 |
| 7 | huEF13n56-Ld-Fc | 95.41 | 93.09 |
| 8 | huEF56n13-Ld-Fc | 95.1 | 91.0 |
| 9 | huEF13n56-Ld-Fc | 96.2 | 91.6 |

| | | | |
|---|---|---|---|
| Conclusion: after 20 days at elevated temperature, the purity was reduced, but within acceptable limits. | | | |

### Example 9: Identification of Functions of huFE Bispecific Antibody-Fc Fusion Protein

### 9.1. Affinity of huFE bispecific antibody-Fc fusion protein

The binding kinetics of the huFE bispecific antibody-Fc fusion proteins obtained in the above example to hApple-Chis and hFXI-Chis proteins were measured by bio-layer interferometry (BLI) using a molecular interaction instrument. The huFE bispecific antibody-Fc fusion proteins obtained in Example 7.2 were diluted to a final concentration of 10 µg/mL and directly immobilized onto an AHC biosensor, and for kinetic measurements, either hApple-Chis protein or hFXI-Chis protein was diluted to 5 concentrations, with a 60-second baseline, binding for 120 seconds, and then dissociation for 900 seconds. The diluent was a kinetic buffer, the regeneration solution was glycine-HCl (pH 1.7), and the neutralization solution was the diluent. The biosensor was Protein A biosensor. The association rate (kon) and dissociation rate (kdis) were calculated using a simple one-to-one Languir binding model (Octet K2 data analysis software version 9.0 (Data analysis 9.0)). The equilibrium dissociation constant (KD) was calculated as the ratio of kdis/kon.

The measured affinity of the huFE bispecific antibody-Fc fusion protein for the hFXI-Chis protein is shown in Table 22 below.

**Table 22**

| | KD (M) |
|---|---|
| huEF13n97-Ld-Fc | 6.70E-10 |
| huEF56n97-Ld-Fc | 6.74E-10 |
| huEF97n13-Ld-Fc | 1.31E-09 |
| huEF97n56-Ld-Fc | 5.51E-10 |
| huEF97n148-Ld-Fc | 4.48E-10 |
| huEF148n13-Ld-Fc | 1.81E-09 |
| huEF13n148-Ld-Fc | 1.93E-09 |
| huEF148n56-Ld-Fc | 1.66E-09 |
| huEF56n148-Ld-Fc | 2.31E-09 |
| huEF148n97-Ld-Fc | 4.64E-10 |
| huEF5n13-Ld-Fc | 1.25E-09 |
| huEF13n5-Ld-Fc | 9.52E-10 |
| huEF5n97-Ld-Fc | 4.50E-10 |
| huEF97n5-Ld-Fc | 2.16E-10 |
| huEF56n5-Ld-Fc | 5.77E-10 |
| huEF5n56-Ld-Fc | 6.11E-10 |
| huEF148n5-Ld-Fc | 1.71E-09 |
| huEF5n148-Ld-Fc | 2.01E-09 |

The measured affinity of the huFE bispecific antibody-Fc fusion protein for the hApple-Chis protein is shown in Table 23 below.

**Table 23**

| | KD (M) |
|---|---|
| huEF13n97-Ld-Fc | <1.0E-12 |
| huEF56n97-Ld-Fc | <1.0E-12 |
| huEF97n13-Ld-Fc | <1.0E-12 |
| huEF97n56-Ld-Fc | <1.0E-12 |
| huEF97n148-Ld-Fc | <1.0E-12 |
| huEF148n13-Ld-Fc | 9.04E-11 |
| huEF13n148-Ld-Fc | <1.0E-12 |
| huEF148n56-Ld-Fc | 1.59E-10 |
| huEF56n148-Ld-Fc | 2.31E-10 |
| huEF148n97-Ld-Fc | <1.0E-12 |
| huEF5n13-Ld-Fc | <1.0E-12 |
| huEF13n5-Ld-Fc | <1.0E-12 |
| huEF5n97-Ld-Fc | <1.0E-12 |
| huEF97n5-Ld-Fc | <1.0E-12 |
| huEF56n5-Ld-Fc | 2.46E-10 |
| huEF5n56-Ld-Fc | 1.11E-10 |
| huEF148n5-Ld-Fc | 4.59E-10 |
| huEF5n148-Ld-Fc | 2.01E-10 |

The above results show that all candidate FXI bispecific antibodies showed good affinities for human FXI factor proteins as well as for human Apple domains and had higher affinities than the parent monospecific antibody.

### 9.2. Specificity of huFE bispecific antibody-Fc fusion protein

The FXI bispecific antibodies obtained in the above examples were selected and the non-specific binding thereof to other coagulation-related proteins was examined by bio-layer interferometry (BLI). The antibody proteins were immobilized onto AHC chips and the proteins examined were commercially available FVII, FIX, FV, FXII, pro-thrombin, α-kallikrein, FVIIa, FIXa, FVa, FXIIa and Thrombin. The experimental results show that all bispecific antibodies did not bind to FVII, FIX, FV, FXII, pro-thrombin, α-kallikrein, FVIIa, FIXa, FVa, FXIIa and Thrombin.

### 9.3. Inhibitory effect of huFE bispecific antibody-Fc fusion protein

### 9.3.1. Inhibitory effect on human FXI activity

The activity of standard human FXI (WHO) was 92%, and the activity of FXI in standard human plasma (purchased from sigma) was 87.5%. The effect of inhibiting the activity of FXI factors in the plasma after adding different single-domain antibody-Fc fusion proteins and controls (14E11, MAA 868-F11) was detected by matching with FXI-deficient plasma. The results of the activity of FXI factors mixed with different antibodies are shown in FIG. 3. The FXI antibodies had relatively good inhibitory activity results against FXI factors and the inhibitory activity results of these antibodies were better than those of control positive antibodies. The 14E11 protein (prepared as above) and MAA868-F11 (prepared according to Patent Application US15/739414) were taken as positive controls.

### 9.3.2. Inhibitory activity against APTT in human whole plasma

The whole blood APTT time was measured after dilution of FXI antibodies to various concentrations in standard human plasma (purchased from Sigma) and incubation at 37 °C for 3 minutes. 14E11 and B1213790-F11a were taken as positive controls.

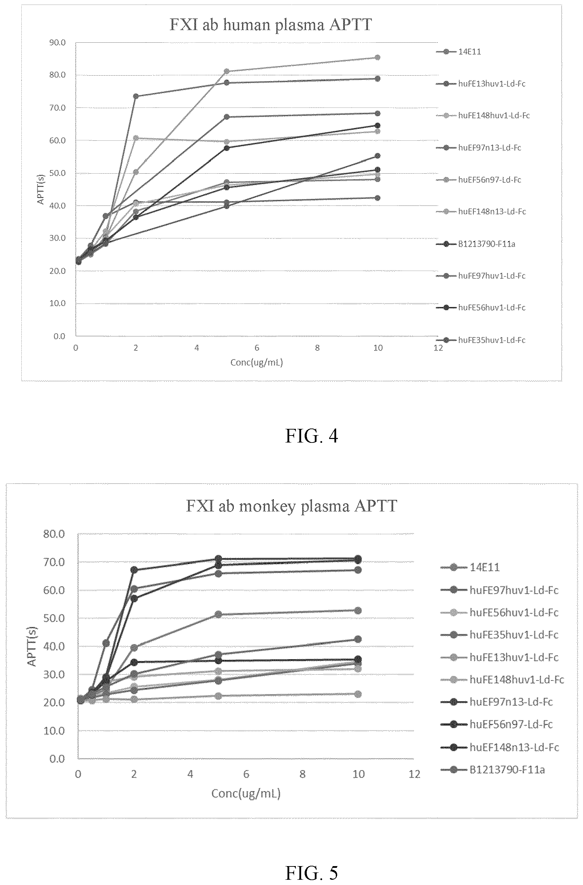

FIG. 4 shows the variations of APTT time with antibody concentration. The results show that all of the candidate FXI antibody proteins can effectively prolong the whole blood APTT coagulation time; and the bispecific antibodies show a better coagulation inhibition effect.

### 9.3.3. Inhibitory activity against APTT of monkey whole plasma

The whole blood APTT time was measured after dilution of FXI antibodies to various concentrations in monkey plasma (purchased from Sigma) and incubation at 37 °C for 3 minutes. 14E11 and B1213790-F11a were taken as positive controls.

The results are shown in FIG. 5.

### 9.3.4. Inhibitory activity against APTT of rabbit whole plasma

The whole blood APTT time was measured by diluting the FXI antibody-Fc fusion proteins and standards to different concentrations with rabbit plasma (purchased from Sigma). 14E11 and B1213790-F11a were taken as positive controls.

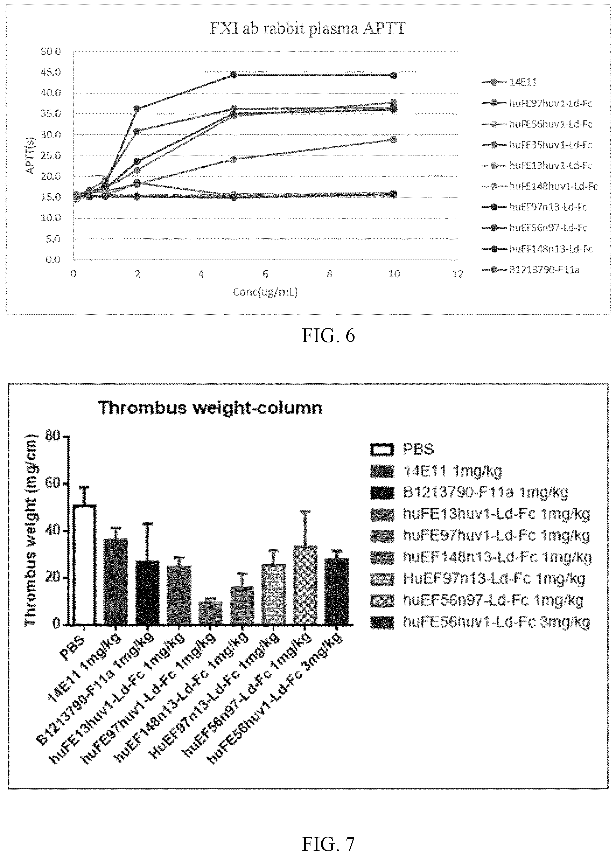

The results are shown in FIG. 6.

The above results show that most FXI candidate antibodies, whether bispecific or not, showed relatively good APTT inhibitory activity in human or monkey plasma. Some antibodies were inactive in rabbits.

The activity of the candidate antibodies in human plasma was substantially better than that of the control positive antibodies. Meanwhile, bispecific antibodies tend to be more active than monospecific antibodies.

### 9.4. Effect of FXI single-domain antibody-Fc fusion proteins and bispecific antibodies on venous thrombosis in rabbit

The rabbits were fasted at night, blood was collected through the ear vein which was subjected to anesthesia by injecting an anaesthetic, ligation was performed at the distal end and the proximal end of the jugular vein, and the interval between two ligatures was uniformly kept at about 3.0 cm. 15 min before molding, 1 mg/kg of the test sample (the huFE single-domain antibody-Fc fusion protein obtained in Example 5.2) or the PBS negative control (see Table 24) was intravenously injected into the ear vein. Artery clamps clamped the proximal end and the distal end of the jugular vein respectively, blood in the blood vessel was completely extracted from the facial vein by using an injector, then 0.3 mL of an agonist with a concentration of 5 mg/mL was injected into a closed section of the blood vessel, the agonist was extracted by using the injector after being incubated for 5 minutes, followed by rinsing with normal saline for 2 times, the artery clamps were loosened to restore the circulation of the blood, and the diameter of the blood vessel was controlled to be 0.8 mm, so as to induce thrombosis. After the restoration of blood circulation for 25 minutes, the distal end and the proximal end of the vein at the closed section were clamped by using artery clamps, the ligated surgical threads at the proximal end and the distal end of the vein were tightened, the closed vein was cut, the thrombus was taken out, and the wet weight of the thrombus was immediately weighed and recorded. After the thrombus was dried in a 60 °C oven for 20 hours, the dry weight of the thrombus was weighed and recorded (see Table 25).

The observation index was thrombus weight, the experimental data were expressed as X±SD, and the significance test was performed using GraphPad Prism 51 way ANOVA (see FIG. 7). Only part of the antibodies showed a good thrombus inhibitory effect under the influence of the species crossability of different antibodies and rabbit species. Monkeys with better species crossability were subsequently selected for the corresponding *in-vivo* activity assay.

**Table 24**

| Group | Number of animals | Test compounds | Dose of administration (mg/kg) | Administration concentration (mg/ml) | Volume of administration (mL/kg) | Solvent | Mode of administration | Frequency of administration |
|---|---|---|---|---|---|---|---|---|
| 1 | 4 | PBS | - | - | 1 | - | IV | Once |
| 2 | 4 | 14E11 | 1 | 1 | 1 | PBS | | |
| 3 | 4 | iFE148-Ld-Fc | | | | | | |
| 4 | 4 | iFES-Ld-Fc | | | | | | |
| 5 | 4 | iFE13-Ld-Fc | | | | | | |
| 6 | 4 | iFE56-Ld-Fc | | | | | | |
| 7 | 4 | iFE97-Ld-Fc | | | | | | |

**Table 25**

| Group | Thrombus weight (mg/cm) | | | | | | | | | |
|---|---|---|---|---|---|---|---|---|---|---|
| PBS | 4.2333 | 55.88333 | 30.88952 | 46.2 | 5.529167 | 5.933333 | 25.21667 | 94.11812 | 25.21667 | 29.78885 |
| 14E11 1mg/kg | 30.71429 | 47.43227 | 55.80357 | 25.49769 | 32.01417 | 25.30662 | | | | |
| B1213790-F11a lmg/kg | 11.03333 | 21.85714 | 73.99537 | 0 | | | | | | |
| huFE13huv 1-Ld-Fc 1mg/kg | 15.75801 | 23.31667 | 36.72667 | 26.00138 | 33.833 | 13.0333 | | | | |
| huFE97huv 1-Ld-Fc 1mg/kg | 5.214286 | 11.53526 | 13.690172 | 2.182882 | 11.99 | 11.39286 | | | | |
| huEF148n13-Ld-Fc 1mg/kg | 3.927249 | 56.43333 | 15.853 | 8.683333 | 5.910667 | 2.914667 | 19.52174 | 12.44524 | | |
| HuEF97n13-Ld-Fc 1mg/kg | 44.71429 | 39.28287 | 11.895 | 35.03179 | 33.353 | 2.594 | 89.06677 | 11.27747 | | |
| huEF56n97-Ld-Fc 1mg/kg | 63.17778 | 191.9768 | 15.02167 | 21.39968 | | | | | | |
| hu56huv 1-Ld-Fc 3mg/kg | 31.36207 | 31.5 | 31.59074 | 17.08769 | | | | | | |

### Sequence information

### Amino acid sequences and corresponding CDR sequences of 23 heavy chain single-domain antibodies

> iFE5

QVQLQESGGGSVQAGGSLRLSCAASGYTYSFNSMGWFRQAPGKEREGVAVIYLGSTHYADSVKG
RFTISQDNAKNMVYLQMNSLKPEDTAMYYCAADLLEYDGTWFGSTYNYWGQGTQVTVSS (SEQ
ID NO: 1)

| | **KABAT** | **AbM** | **Chothia** | **IMGT** |
|---|---|---|---|---|
| CDR1 | FNSMG(SEQ ID NO:24) | GYTYSFNSMG(SEQ ID NO:27) | GYTYSFN(SEQ ID NO:30) | GYTYSFNS(SEQ ID NO:33) |
| CDR2 | IYLGSTHYADSVKG( SEQ ID NO:25) | IYLGSTU(SEQ ID NO:28) | LGS(SEQ ID NO:31) | YLGST(SEQ ID NO:34) |
| CDR3 | DLLEYDGTWFGSTY NY(SEQ ID NO:26) | DLLEYDGTWFGSTY NY(SEQ ID NO:29) | DLLEYDGTWFGSTY NY(SEQ ID NO:32) | AADLLEYDGTWFGSTY NY(SEQ ID NO:35) |

> iFE7m

QVQLQESGGGLVQPGGSLRLSCAASGLTFSLYDMSWVRQAPGKGLEGVSGINSGDGSTYYADSVK
GRFTISRDNAKNTLYLQMNSLKTEDTAVYYCATGRTRETYFREDDWGQGTQVTVSS (SEQ ID NO:
2)

| | **KABAT** | **AbM** | **Chothia** | **IMGT** |
|---|---|---|---|---|
| CDR1 | LYDMS(SEQ ID NO:36) | GLTFSLYDMS(SEQ ID NO:39) | NSGDGS(SEQ ID NO:42) | GLTFSLYD(SEQ ID NO:45) |
| CDR2 | GINSGDGSTYYADSVKG(SE Q ID NO:37) | GINSGDGSTY(SEQ ID NO:40) | NSGDGS(SEQ ID NO:43) | INSGDGST(SEQ ID NO:46) |
| CDR3 | GRTRETYFREDD(SEQ ID NO:38) | GRTRETYFREDD(SE Q ID NO:41) | GRTRETYFREDD(SE Q ID NO:44) | ATGRTRETYFREDD( SEQ ID NO:47) |

> iFE11m

QVQLQESGGGLVQPGGSLRLSCAASGLRFSVYDMSWVRQAPGKGLEGVSGIDSGGGSTYYADSV
KGRFTISRDDAKNTLYLQMNSLKTEDTAVYYCATGLGGSWYREPDWGQGTQVTVSS (SEQ ID NO:
3)

| | **KABAT** | **AbM** | **Chothia** | **IMGT** |
|---|---|---|---|---|
| CDR1 | VYDMS(SEQ ID NO:48) | GLRFSVYDMS(SEQ ID NO:51) | GLRFSVY(SEQ ID NO:54) | GLRFSVYD(SEQ ID NO:57) |
| CDR2 | GIDSGGGSTYYADSVKG(SE Q ID NO:49) | GIDSGGGSTY(SEQ ID NO:52) | DSGGGS(SEQ ID NO:55) | IDSGGGST(SEQ ID NO:58) |
| CDR3 | GLGGSWYREPD(SEQ ID NO:50) | GLGGSWYREPD(SE Q ID NO:53) | GLGGSWYREPD(SE Q ID NO:56) | ATGLGGSWYREPD( SEQ ID NO:59) |

> iFE13

QVQLQESGGGSVQAGGSLRLSCAGDGYTYSMGAMAWFRQAPGKEREGVALIATSSGFISYTDSV
KGRFTISQDDAKIYLQMNSLKPEDTAMYYCAAGTQFSWSPGSYNYWGQGTQVTVSS (SEQ ID
NO: 4)

| | **KABAT** | **AbM** | **Chothia** | **IMGT** |
|---|---|---|---|---|
| CDR1 | MGAMA(SEQ ID NO:60) | GYTYSMGAMA(SE Q ID NO:63) | GYTYSMG(SEQ ID NO:66) | GYTYSMGA(SEQ ID NO: 69) |
| CDR2 | LIATSSGFISYTDSVK G(SEQ ID NO:61) | LIATSSGFIS(SEQ ID NO: 64) | ATSSGF(SEQ ID NO:67) | IATSSGFI(SEQ ID NO: 70) |
| CDR3 | GTQFSWSPGSYNY(S EQ ID NO:62) | GTQFSWSPGSYNY( SEQ ID NO:65) | GTQFSWSPGSYNY( SEQ ID NO:68) | AAGTQFSWSPGSYNY( SEQ ID NO:71) |

> iFE15m

QVQLQESGGGLVQPGGSLRLSCAASGFTFSNYDMSWVRQAPGKGLEGVSGINSGGGHPHYADSV
KGRFTISRDNAKNTLYLQMNSLKTEDTAVYFCATDEGERYGEDFGYWGQGTQVTVSS (SEQ ID
NO: 5)

| | **KABAT** | **AbM** | **Chothia** | **IMGT** |
|---|---|---|---|---|
| CDR1 | NYDMS(SEQ ID NO:72) | GFTFSNYDMS(SEQ ID NO:75) | GFTFSNY(SEQ ID NO:78) | GFTFSNYD(SEQ ID NO:81) |
| CDR2 | GINSGGGHPHYADSVKG(S EQ ID NO:73) | GINSGGGHPH(SEQ ID NO:76) | NSGGGH(SEQ ID NO: 79) | INSGGGHP(SEQ ID NO:82) |
| CDR3 | DEGERYGEDFGY(SEQ ID NO:74) | DEGERYGEDFGY(S EQ ID NO:77) | DEGERYGEDFGY(S EQ ID NO:80) | ATDEGERYGEDFGY (SEQ ID NO:83) |

> iFE17EREG

QVQLQESGGGLVQPGGSLRVSCAASGFTFSLYDMSWVRQAPGKEREGVSGINSDDGSTSYADSVK
GRFTISRDNAKNTLYLQMNSLKTEDTAVYYCATGVGARWFREDDWGQGTQVTVSS (SEQ ID NO:
6)

| | **KABAT** | **AbM** | **Chothia** | **IMGT** |
|---|---|---|---|---|
| CDR1 | NYDMS(SEQ ID NO:84) | GFTFSNYDMS(SEQ ID NO:87) | GFTFSNY(SEQ ID NO:90) | GFTFSNYD(SEQ ID NO:93) |
| CDR2 | GINSGGGHPHYADSVKG(S EQ ID NO:85) | GINSGGGHPH(SEQ ID NO:88) | NSGGGH(SEQ ID NO:91) | INSGGGHP(SEQ ID NO:94) |
| CDR3 | DEGERYGEDFGY(SEQ ID NO:86) | DEGERYGEDFGY(S EQ ID NO:89) | DEGERYGEDFGY(S EQ ID NO:92) | ATDEGERYGEDFGY (SEQ ID NO:95) |

> iFE22m

QVQLQESGGGLVQPGGSLRLSCAASGLRFEIYDMSWVRQAPGKGLEGVSAINSGGGSTYYADSV
KGRFTISRDDVKNTLYLQMNSLKTEDTAVYYCATGDGGSWYRDGDWGQGTQVTVSS (SEQ ID
NO: 7)

| | **KABAT** | **AbM** | **Chothia** | **IMGT** |
|---|---|---|---|---|
| CDR1 | IYDMS(SEQ ID NO:96) | GLRFEIYDMS(SEQ ID NO:99) | GLRFEIY(SEQ ID NO: 102) | GLRFEIYD(SEQ ID NO:105) |
| CDR2 | AINSGGGSTYYADSVKG(SE Q ID NO:97) | AINSGGGSTY(SEQ ID NO:100) | NSGGGS(SEQ ID NO:103) | INSGGGST(SEQ ID NO:106) |
| CDR3 | GDGGSWYRDGD(SEQ ID NO:98) | GDGGSWYRDGD(S EQ ID NO:101) | GDGGSWYRDGD(S EQ ID NO:104) | ATGDGGSWYRDGD (SEQ ID NO:107) |

> iFE29m

QVQLQESGGGLVQPGGSLRLSCVASGLTFSLYDMSWVRQAPGKGLEGVSGISGESGGSTYYADSV
KGRFTISRDNAKNTLYLQMNSLKTEDTAVYYCATGDRGTWYREDDWGQGTQVTVSS (SEQ ID
NO: 8)

| | **KABAT** | **AbM** | **Chothia** | **IMGT** |
|---|---|---|---|---|
| CDR1 | LYDMS(SEQ ID NO:108) | GLTFSLYDMS(SEQ ID NO:111) | GLTFSLY(SEQ ID NO:114) | GLTFSLYD(SEQ ID NO:117) |
| CDR2 | GISGESGGSTYYADSVKG(S EQ ID NO:109) | GISGESGGSTY(SEQ ID NO:112) | SGESGGS(SEQ ID NO:115) | ISGESGGST(SEQ ID NO:118) |
| CDR3 | GDRGTWYREDD(SEQ ID | GDRGTWYREDD(SE | GDRGTWYREDD(SE | ATGDRGTWYREDD( |
| | NO:110) | Q ID NO:113) | Q ID NO: 116) | SEQ ID NO:119) |

> iFE30m

QVQLQESGGGLVQPGGSLRLSCAASGLRFSIYDMSWVRQAPGKGLEGVSGINSGGGSTYYADSVK
GRFTISRDNAKNTLYLQMNSLKTEDTAVYYCATGLGGSWFREDDWGQGTQVTVSS (SEQ ID NO:
9)

| | **KABAT** | **AbM** | **Chothia** | **IMGT** |
|---|---|---|---|---|
| CDR1 | IYDMS(SEQ ID NO:120) | GLRFSIYDMS(SEQ ID NO:123) | GLRFSIY(SEQ ID NO:126) | GLRFSIYD(SEQ ID NO:129) |
| CDR2 | GINSGGGSTYYADSVKG(SE Q ID NO:121) | GINSGGGSTY(SEQ ID NO:124) | NSGGGS(SEQ ID NO:127) | INSGGGST(SEQ ID NO:130) |
| CDR3 | GLGGSWFREDD(SEQ ID NO:122) | GLGGSWFREDD(SE Q ID NO:125) | GLGGSWFREDD(SE Q ID NO:128) | ATGLGGSWFREDD( SEQ ID NO:131) |

> iFE35

QVQLQESGGGSVQAGGSLGLSCTASGFTFDDSDMGWYRQAPGNECELVSTISSDGRGFYADSVKG
RFTISQDRAKNTVYLHMNSLKPEDTAVYYCAAGWYGSFCSGLGQGTQVTVSS (SEQ ID NO: 10)

| | **KABAT** | **AbM** | **Chothia** | **IMGT** |
|---|---|---|---|---|
| CDR1 | DSDMG(SEQ ID NO:132) | GFTFDDSDMG(SEQ ID NO:135) | GFTFDDS(SEQ ID NO:138) | GFTFDDSD(SEQ ID NO:141) |
| CDR2 | TISSDGRGFYADSVKG(SEQ ID NO:133) | TISSDGRGF(SEQ ID NO:136) | SSDGR(SEQ ID NO: 139) | ISSDGRG(SEQ ID NO: 142) |
| CDR3 | GWYGSFCSG(SEQ ID NO: 134) | GWYGSFCSG(SEQ ID NO:137) | GWYGSFCSG(SEQ ID NO:140) | AAGWYGSFCSG(SE Q ID NO:143) |

> iFE43

QVQLQESGGGSVQAGGSLRLSCAASGLRFSIYDWSWVRQAPGKGLEWVSAINSGGGVTSYADSV
KGRVTISRDNAKNTLYLQMHSLKTEDTAVYYCATGDGGSWYRDGDWGQGTQVTVSS (SEQ ID
NO: 11)

| | **KABAT** | **AbM** | **Chothia** | **IMGT** |
|---|---|---|---|---|
| CDR1 | IYDWS(SEQ ID NO:144) | GLRFSIYDWS(SEQ ID NO:147) | GLRFSIY(SEQ ID NO:150) | GLRFSIYD(SEQ ID NO:153) |
| CDR2 | AINSGGGVTSYADSVKG(S EQ ID NO:145) | AINSGGGVTS(SEQ ID NO:148) | NSGGGV(SEQ ID NO:151) | GLRFSIYD(SEQ ID NO:154) |
| CDR3 | GDGGSWYRDGD(SEQ ID NO: 146) | GDGGSWYRDGD(S EQ ID NO:149) | GDGGSWYRDGD(SE Q ID NO:152) | ATGDGGSWYRDGD( SEQ ID NO:155) |

> iFE49

QVQLQESGGDSVQAGGSLRLSCAASGLISTGLISTRHCMSWFRQAPGKEREEVAVIGSDGSTRYGD

FAKGRFTISKDTAKNTLYLQMNSLEPEDTAMYYCAAGPLVSTASRRRTRCPEGPRENIYWGQGTQ
VTVSS (SEQ ID NO: 12)

| | **KABAT** | **AbM** | **Chothia** | **IMGT** |
|---|---|---|---|---|
| CDR1 | GLISTRHCMS(SEQ ID NO:156) | GLISTGLISTRHCMS( SEQ ID NO:159) | GLISTGLISTRH(SEQ ID NO:162) | GLISTGLISTRHC(SEQ ID NO:165) |
| CDR2 | VIGSDGSTRYGDFA KG(SEQ ID NO:157) | VIGSDGSTR(SEQ ID NO:160) | GSDGS(SEQ ID NO:163) | IGSDGST(SEQ ID NO:166) |
| CDR3 | GPLVSTASRRRTRC PEGPRENIY(SEQ ID NO:158) | GPLVSTASRRRTRCP EGPRENIY(SEQ ID NO:161) | GPLVSTASRRRTRCP EGPRENIY(SEQ ID NO:163) | AAGPLVSTASRRRTRCP EGPRENIY(SEQ ID NO:167) |

> iFE50

QVQLQESGGGLVQPGGSLRLSCAASGFTFSLYDMSWVRQAPGQGLEWVSAITNPGGSTYYADSV
KDRFTISRDNAKNTLYLQMNSLKTEDTAVYYCAIGRRATFQREKDWGQGTQVTVSS (SEQ ID NO:
13)

| | **KABAT** | **AbM** | **Chothia** | **IMGT** |
|---|---|---|---|---|
| CDR1 | LYDMS(SEQ ID NO:168) | GFTFSLYDMS(SEQ ID NO:171) | GFTFSLY(SEQ ID NO:174) | GFTFSLYD(SEQ ID NO:177) |
| CDR2 | AITNPGGSTYYADSVKD(S EQ ID NO:169) | AITNPGGSTY(SEQ ID NO:172) | TNPGGS(SEQ ID NO:175) | ITNPGGST(SEQ ID NO:178) |
| CDR3 | GRRATFQREKD(SEQ ID NO:170) | GRRATFQREKD(SE Q ID NO:173) | GRRATFQREKD(SE Q ID NO:176) | AIGRRATFQREKD( SEQ ID NO:179) |

> iFE56

QVQLQESGGGSVQAGGSLRLSCAASGHTYSSNYCMAWFRQAPGKEREGVAAIYSDGSTSYADSV
KGRFTISKDNAKNTLYLQIDSLKPEDTSLYYCAATAYEGSWTGKQPLCLLYEYTYWGQGTQVTVS
S (SEQ ID NO: 14)

| | **KABAT** | **AbM** | **Chothia** | **IMGT** |
|---|---|---|---|---|
| CDR1 | NYCMA(SEQ ID NO:180) | GHTYSSNYCMA(SEQ ID NO:183) | GHTYSSNY(SEQ ID NO:186) | GHTYSSNYC(S EQ ID NO:189) |
| CDR2 | AIYSDGSTSYADSVKG(S EQ ID NO:181) | AIYSDGSTS(SEQ ID NO:184) | YSDGST(SEQ ID NO:187) | IYSDGST(SEQ ID NO:190) |
| CDR3 | TAYEGSWTGKQPLCLLY EYTY(SEQ ID NO:182) | TAYEGSWTGKQPLCLLY EYTY(SEQ ID NO:185) | TAYEGSWTGKQPLCLLY EYTY(SEQ ID NO:188) | AATAYEGSWT GKQPLCLLYE YTY(SEQ ID NO:191) |

> iFE70m

QVQLQESGGGLVQPGGSLRLSCAASGFTFSLYDMSWVRQAPGKGLDGVSGIGGDGGSTYYADSV
KGRFAISRDDAKNTLYLQMNSLKIEDTAVYYCALGRPPYTDYDSRGQGTQVTVSS (SEQ ID NO:

15)

| | **KABAT** | **AbM** | **Chothia** | **IMGT** |
|---|---|---|---|---|
| CDR1 | LYDMS(SEQ ID NO:192) | GFTFSLYDMS(SEQ ID NO:195) | GFTFSLY(SEQ ID NO:198) | GFTFSLYD(SEQ ID NO:201) |
| CDR2 | GIGGDGGSTYYADSVKG(S EQ ID NO:193) | GIGGDGGSTY(SEQ ID NO:196) | GGDGGS(SEQ ID NO: 199) | IGGDGGST(SEQ ID NO:202) |
| CDR3 | GRPPYTDYDS(SEQ ID NO: 194) | GRPPYTDYDS(SEQ ID NO:197) | GRPPYTDYDS(SEQ ID NO:200) | ALGRPPYTDYDS(SE Q ID NO:203) |

> iFE96

QVQLQESGGGSVQAGGSLRLSCAASGFTASSNFMGWFRQAPGKEREGVAAIYTGGASTFYADSV
KGRFTISQDNAKNTVYLEMDTLKPEDTAMYYCAAAPRWIAPLRADRYEYWGQGTQVTVSS (SEQ
ID NO: 16)

| | **KABAT** | **AbM** | **Chothia** | **IMGT** |
|---|---|---|---|---|
| CDR1 | SNFMG(SEQ ID NO:204) | GFTASSNFMG(SEQ ID NO:207) | GFTASSN(SEQ ID NO:210) | GFTASSNF(SEQ ID NO:213) |
| CDR2 | AIYTGGASTFYADSVKG(S EQ ID NO:205) | AIYTGGASTF(SEQ ID NO:208) | YTGGAS(SEQ ID NO:211) | IYTGGAST(SEQ ID NO:214) |
| CDR3 | APRWIAPLRADRYEY(SEQ ID NO:206) | APRWIAPLRADRYE Y(SEQ ID NO:209) | APRWIAPLRADRYE Y(SEQ ID NO:212) | AAAPRWIAPLRADR YEY(SEQ ID NO:215) |

> iFE97

QVQLQESGGGSVQAGGSLRLSCTASEFTFDDSDMAWYRQAPGNECELVSTITSDGGTYYADSVKG
RFTISQDNAKNTMYLQMNNLKPEDTAVYYCAADQWGSAEGDCTSSYPGGYWGQGTQVTVSS
(SEQ ID NO: 17)

| | **KABAT** | **AbM** | **Chothia** | **IMGT** |
|---|---|---|---|---|
| CDR1 | DSDMA(SEQ ID NO:216) | EFTFDDSDMA(SEQ ID NO:219) | EFTFDDS(SEQ ID NO:222) | EFTFDDSD(SEQ ID NO:225) |
| CDR2 | TITSDGGTYYADSVKG(SE Q ID NO:217) | TITSDGGTY(SEQ ID NO:220) | TSDGG(SEQ ID NO:223) | ITSDGGT(SEQ ID NO:226) |
| CDR3 | DQWGSAEGDCTSSYPGGY (SEQ ID NO:218) | DQWGSAEGDCTSS YPGGY(SEQ ID NO:221) | DQWGSAEGDCTSS YPGGY(SEQ ID NO:224) | AADQWGSAEGDCT SSYPGGY(SEQ ID NO:227) |

> iFE107m

QVQLQESGGGLVQPGGSLRLSCAASGFTFSLYDMSWVRQSPGKGLEGVSAIDSGDGRTYYADSVK
GRFTISRDDAKNTLYLQMNSLKTGDTAVYYCANGGRGSWFRESDWGQGTQVTVSS (SEQ ID NO:
18)

| | **KABAT** | **AbM** | **Chothia** | **IMGT** |
|---|---|---|---|---|
| CDR1 | LYDMS(SEQ ID NO:228) | GFTFSLYDMS(SE Q ID NO:231) | GFTFSLY(SEQ ID NO:234) | GFTFSLYD(SEQ ID NO:237) |
| CDR2 | AIDSGDGRTYYADSVKG( SEQ ID NO:229) | AIDSGDGRTY(SE Q ID NO:232) | DSGDGR(SEQ ID NO:235) | IDSGDGRT(SEQ ID NO:238) |
| CDR3 | GGRGSWFRESD(SEQ ID NO:230) | GGRGSWFRESD( SEQ ID NO:233) | GGRGSWFRESD( SEQ ID NO:236) | ANGGRGSWFRESD( SEQ ID NO:239) |

> iFE128m

QVQLQESGGRLVQPGGSLRLSCVASGFTFEYYDMSWVRQAPGKGLEGVSAIDARSITDYADAVKG
RFTISRDNAKNTLYLQLNNLKTEDTAMYYCANGGRIGWFREDDWGQGTQVTVSS (SEQ ID NO:
19)

| | **KABAT** | **AbM** | **Chothia** | **IMGT** |
|---|---|---|---|---|
| CDR1 | YYDMS(SEQ ID NO:240) | GFTFEYYDMS(SEQ ID NO:243) | GFTFEYY(SEQ ID NO:246) | GFTFEYYD(SEQ ID NO:249) |
| CDR2 | AIDARSITDYADAVKG(SEQ ID NO:241) | AIDARSITD(SEQ ID NO:244) | DARSI(SEQ ID NO:247) | IDARSIT(SEQ ID NO:250) |
| CDR3 | GGRIGWFREDD(SEQ ID NO:242) | GGRIGWFREDD(SE Q ID NO:245) | GGRIGWFREDD(SE Q ID NO:248) | ANGGRIGWFREDD (SEQ ID NO:251) |

> iFE148

QVQLQESGGGSVQAGGSLRLSCVASGYTFNNNFVGWFRQAPGKEREGVAAIYSLGGSTYYADSV
KGRFTISQDNAKNTVYLQMDSLKPEDTATYYCAGAITWVPPLSHRRYTYWGQGTQVTVSS (SEQ
ID NO: 20)

| | **KABAT** | **AbM** | **Chothia** | **IMGT** |
|---|---|---|---|---|
| CDR 1 | NNFVG(SEQ ID NO:252) | GYTFNNNFVG(SEQ ID NO:255) | GYTFNNN(SEQ ID NO:258) | GYTFNNNF(SEQ ID NO:261) |
| CDR 2 | AIYSLGGSTYYADSVK G(SEQ ID NO:253) | AIYSLGGSTY(SEQ ID NO:256) | YSLGGS(SEQ ID NO:258) | IYSLGGST(SEQ ID NO:263) |
| CDR 3 | AITWVPPLSHRRYTY(S EQ ID NO:254) | AITWVPPLSHRRYTY (SEQ ID NO:257) | AITWVPPLSHRRYTY (SEQ ID NO:260) | AGAITWVPPLSHRRYT Y(SEQ ID NO:263) |

> iFE163

QVQLQESGGDSVQAGGSLRLSCIASGFTFDDSDMGWYRQAPGHQCEFVSHITNDGTTYYADSVK
GRFTISRDNAKNTVYLQMNSLKPEDAAVYYCAADAWGGGRSTCVNPIGYWGQGTQVTVSS (SEQ
ID NO: 21)

| | **KABAT** | **AbM** | **Chothia** | **IMGT** |
|---|---|---|---|---|
| CDR1 | DSDMG(SEQ ID NO:264) | GFTFDDSDMG(SEQ ID NO:267) | GFTFDDS(SEQ ID NO:270) | GFTFDDSD(SEQ ID NO:273) |
| CDR2 | HITNDGTTYYADSVK G(SEQ ID NO:265) | HITNDGTTY(SEQ ID NO:268) | TNDGT(SEQ ID NO:271) | ITNDGTT(SEQ ID NO:274) |
| CDR3 | DAWGGGRSTCVNPI GY(SEQ ID NO:266) | DAWGGGRSTCVNPIG Y(SEQ ID NO:269) | DAWGGGRSTCVNPIG Y(SEQ ID NO:272) | AADAWGGGRSTCVNPI GY(SEQ ID NO:275) |

> iFE166

QVQLQESGGGSVQAGGSLRLSCVASGFTGCRNIMSWYRQAPGKDREFVSAIDSDGVDYADSVKG
RFSISQHNDKKTMYLQMTSLKLEDTAMYYCRRVDFGTTSQCRGNYWGQGTQVTVSS (SEQ ID
NO: 22)

| | **KABAT** | **AbM** | **Chothia** | **IMGT** |
|---|---|---|---|---|
| CDR 1 | RNIMS(SEQ ID NO:276) | GFTGCRNIMS(SEQ ID NO:279) | GFTGCRN(SEQ ID NO:282) | GFTGCRNI(SEQ ID NO:285) |
| CDR 2 | AIDSDGVDYADSVKG(S EQ ID NO:277) | AIDSDGVD(SEQ ID NO:280) | DSDG(SEQ ID NO:283) | IDSDGV(SEQ ID NO:286) |
| CDR 3 | VDFGTTSQCRGNY(SE Q ID NO:278) | VDFGTTSQCRGNY(S EQ ID NO:281) | VDFGTTSQCRGNY(S EQ ID NO:284) | RRVDFGTTSQCRGNY(S EQ ID NO:287) |

> iFE168

QVQLQESGGGSVQAGGSLRLSCAASGYTFSNNFVGWFRQAPGKKREGVATIYTLGSSTYYADSVK
GRFTISQDNAKNTLYLEMNSLKPEDTAMYYCAAARGWVSPLDPARFVYWGQGTQVTVSS (SEQ
ID NO: 23)

| | **KABAT** | **AbM** | **Chothia** | **IMGT** |
|---|---|---|---|---|
| CDR1 | NNFVG(SEQ ID NO:288) | GYTFSNNFVG(SEQ ID NO:291) | GYTFSNN(SEQ ID NO:294) | GYTFSNNF(SEQ ID NO:297) |
| CDR2 | TIYTLGSSTYYADSV KG(SEQ ID NO:289) | TIYTLGSSTY(SEQ ID NO:292) | YTLGSS(SEQ ID NO:295) | IYTLGSST(SEQ ID NO:298) |
| CDR3 | ARGWVSPLDPARFV Y(SEQ ID NO:290) | ARGWVSPLDPARFV Y(SEQ ID NO:293) | ARGWVSPLDPARFV Y(SEQ ID NO:296) | AAARGWVSPLDPARFV Y(SEQ ID NO:299) |

> huFE5huv1 
QVQLVESGGGSVQAGGSLRLSCAASGYTYSFNSMGWFRQAPGKEREGVAVIYLGSTHYADSVK
GRFTISQDNSKNTVYLQMNSLRAEDTAVYYCAADLLEYDGTWFGSTYNYWGQGTLVTVSS (SEQ
ID NO: 300)
> huFE5huv2 
QVQLVESGGGLVQPGGSLRLSCAASGYTYSFNSMGWFRQAPGKGLEGVSVIYLGSTHYADSVKG
RFTISRDNSKNTLYLQMNSLRAEDTAVYYCAADLLEYDGTWFGSTYNYWGQGTLVTVSS (SEQ
ID NO: 301)
> huFE5huv3 
QVQLVESGGGLVQPGGSLRLSCAASGYTYSFNSMGWFRQAPGKEREGVAVIYLGSTHYADSVK
GRFTISQDNSKNTVYLQMNSLRAEDTAVYYCAADLLEYDGTWFGSTYNYWGQGTLVTVSS (SEQ
ID NO: 302)
> huFE5huv4 
QVQLVESGGGSVQAGGSLRLSCAASGYTYSFNSMGWFRQAPGKGLEGVSVIYLGSTHYADSVKG
RFTISQDNSKNTVYLQMNSLRAEDTAVYYCAADLLEYDGTWFGSTYNYWGQGTLVTVSS (SEQ
ID NO: 303)
> huFE5huv5 
QVQLVESGGGSVQAGGSLRLSCAASGYTYSFNSMGWFRQAPGKGLEGVSVIYLGSTHYADSVKG
RFTISQDNSKNTLYLQMNSLKPEDTAVYYCAADLLEYDGTWFGSTYNYWGQGTLVTVSS (SEQ
ID NO: 304)
> huFE5huv6 
QVQLVESGGGLVQPGGSLRLSCAASGYTYSFNSMGWFRQAPGKGLEGVSVIYLGSTHYADSVKG
RFTISQDNSKNTLYLQMNSLKPEDTAVYYCAADLLEYDGTWFGSTYNYWGQGTLVTVSS (SEQ
ID NO: 305)
> huFE13huv1 
QVQLVESGGGSVQAGGSLRLSCAASGYTYSMGAMAWFRQAPGKEREGVALIATSSGFISYTDSV
KGRFTISQDNSKNTVYLQMNSLRAEDTAVYYCAAGTQFSWSPGSYNYWGQGTLVTVSS (SEQ ID
NO: 306)
> huFE13huv2 
QVQLVESGGGLVQPGGSLRLSCAASGYTYSMGAMAWFRQAPGKGLEGVSLIATSSGFISYTDSV
KGRFTISRDNSKNTLYLQMNSLRAEDTAVYYCAAGTQFSWSPGSYNYWGQGTLVTVSS (SEQ ID
NO: 307)
> huFE13huv3 
QVQLVESGGGLVQPGGSLRLSCAASGYTYSMGAMAWFRQAPGKEREGVALIATSSGFISYTDSV
KGRFTISQDNSKNTVYLQMNSLRAEDTAVYYCAAGTQFSWSPGSYNYWGQGTLVTVSS (SEQ ID
NO: 308)
> huFE13huv4 
QVQLVESGGGSVQAGGSLRLSCAASGYTYSMGAMAWFRQAPGKGLEGVSLIATSSGFISYTDSV
KGRFTISQDNSKNTVYLQMNSLRAEDTAVYYCAAGTQFSWSPGSYNYWGQGTLVTVSS (SEQ ID
NO: 309)
> huFE13huv5 
QVQLVESGGGSVQAGGSLRLSCAASGYTYSMGAMAWFRQAPGKGLEGVSLIATSSGFISYTDSV
KGRFTISQDNSKNTLYLQMNSLKPEDTAVYYCAAGTQFSWSPGSYNYWGQGTLVTVSS (SEQ ID
NO: 310)
> huFE13huv6 
QVQLVESGGGLVQPGGSLRLSCAASGYTYSMGAMAWFRQAPGKGLEGVSLIATSSGFISYTDSV
KGRFTISQDNSKNTLYLQMNSLKPEDTAVYYCAAGTQFSWSPGSYNYWGQGTLVTVSS (SEQ ID
NO: 311)
> huFE35huv1 
QVQLVESGGGSVQAGGSLRLSCTASGFTFDDSDMGWYRQAPGNECELVSTISSDGRGFYADSVK
GRFTISQDNSKNTVYLQMNSLRAEDTAVYYCAAGWYGSFCSGLGQGTLVTVSS (SEQ ID NO: 312)
> huFE35huv2 
QVQLVESGGGLVQPGGSLRLSCAASGFTFDDSDMGWYRQAPGNECELVSTISSDGRGFYADSVK
GRFTISRDNSKNTLYLQMNSLRAEDTAVYYCAAGWYGSFCSGLGQGTLVTVSS (SEQ ID NO: 313)
> huFE35huv3 
QVQLVESGGGLVQPGGSLRLSCTASGFTFDDSDMGWYRQAPGNECELVSTISSDGRGFYADSVK
GRFTISRDNSKNTVYLQMNSLRAEDTAVYYCAAGWYGSFCSGLGQGTLVTVSS (SEQ ID NO: 314)
> huFE35huv4 
QVQLVESGGGSVQAGGSLRLSCTASGFTFDDSDMGWYRQAPGNECELVSTISSDGRGFYADSVK
GRFTISQDNSKNTVYLQMNSLRAEDTAVYYCAAGWYGSFCSGLGQGTLVTVSS (SEQ ID NO: 315)
> huFE35huv5 
QVQLVESGGGSVQAGGSLRLSCTASGFTFDDSDMGWYRQAPGNECELVSTISSDGRGFYADSVK
GRFTISQDNSKNTLYLQMNSLKPEDTAVYYCAAGWYGSFCSGLGQGTLVTVSS (SEQ ID NO: 316)
> huFE35huv6 
QVQLVESGGGLVQPGGSLRLSCTASGFTFDDSDMGWYRQAPGNECELVSTISSDGRGFYADSVK
GRFTISRDNSKNTLYLQMNSLKPEDTAVYYCAAGWYGSFCSGLGQGTLVTVSS (SEQ ID NO: 317)
> huFE56huv1 
QVQLVESGGGSVQAGGSLRLSCAASGHTYSSNYCMAWFRQAPGKEREGVAAIYSDGSTSYADSV
KGRFTISKDNSKNTLYLQMNSLRAEDTAVYYCAATAYEGSWTGKQPLCLLYEYTYWGQGTLVTV
SS (SEQ ID NO: 318)
> huFE56huv2 
QVQLVESGGGLVQPGGSLRLSCAASGHTYSSNYCMAWFRQAPGKEREGVAAIYSDGSTSYADSV
KGRFTISRDNSKNTLYLQMNSLRAEDTAVYYCAATAYEGSWTGKQPLCLLYEYTYWGQGTLVTV
SS (SEQ ID NO: 319)
> huFE56huv3 
QVQLVESGGGLVQPGGSLRLSCAASGHTYSSNYCMAWFRQAPGKEREGVAAIYSDGSTSYADSV
KGRFTISKDNSKNTLYLQMNSLRAEDTAVYYCAATAYEGSWTGKQPLCLLYEYTYWGQGTLVTV
SS (SEQ ID NO: 320)
> huFE56huv4 
QVQLVESGGGSVQAGGSLRLSCAASGHTYSSNYCMAWFRQAPGKGLEGVSAIYSDGSTSYADSV
KGRFTISKDNSKNTLYLQMNSLRAEDTAVYYCAATAYEGSWTGKQPLCLLYEYTYWGQGTLVTV
SS (SEQ ID NO: 321)
> huFE56huv5 
QVQLVESGGGSVQAGGSLRLSCAASGHTYSSNYCMAWFRQAPGKGLEGVSAIYSDGSTSYADSV
KGRFTISKDNSKNTLYLQMNSLKPEDTAVYYCAATAYEGSWTGKQPLCLLYEYTYWGQGTLVTV
SS (SEQ ID NO: 322)
> huFE56huv6 
QVQLVESGGGLVQPGGSLRLSCAASGHTYSSNYCMAWFRQAPGKGLEGVSAIYSDGSTSYADSV
KGRFTISKDNSKNTLYLQMNSLKPEDTAVYYCAATAYEGSWTGKQPLCLLYEYTYWGQGTLVTV
SS (SEQ ID NO: 323)
> huFE97huv1 
QVQLVESGGGSVQAGGSLRLSCTASEFTFDDSDMAWYRQAPGNECELVSTITSDGGTYYADSVK
GRFTISQDNSKNTMYLQMNSLRAEDTAVYYCAADQWGSAEGDCTSSYPGGYWGQGTLVTVSS
(SEQ ID NO: 324)
> huFE97huv2 
QVQLVESGGGLVQPGGSLRLSCAASEFTFDDSDMAWYRQAPGNECELVSTITSDGGTYYADSVK
GRFTISRDNSKNTLYLQMNSLRAEDTAVYYCAADQWGSAEGDCTSSYPGGYWGQGTLVTVSS
(SEQ ID NO: 325)
> huFE97huv3 
QVQLVESGGGLVQPGGSLRLSCTASEFTFDDSDMAWYRQAPGNECELVSTITSDGGTYYADSVK
GRFTISQDNSKNTMYLQMNSLRAEDTAVYYCAADQWGSAEGDCTSSYPGGYWGQGTLVTVSS
(SEQ ID NO: 326)
> huFE97huv4 QVQLVESGGGSVQAGGSLRLSCTASEFTFDDSDMAWYRQAPGNECELVSTITSDGGTYYADSVK 
GRFTISRDNSKNTMYLQMNSLRAEDTAVYYCAADQWGSAEGDCTSSYPGGYWGQGTLVTVSS
(SEQ ID NO: 327)
> huFE97huv5 
QVQLVESGGGSVQAGGSLRLSCTASEFTFDDSDMAWYRQAPGNECELVSTITSDGGTYYADSVK
GRFTISRDNSKNTLYLQMNSLKPEDTAVYYCAADQWGSAEGDCTSSYPGGYWGQGTLVTVSS
(SEQ ID NO: 328)
> huFE97huv6 
QVQLVESGGGLVQPGGSLRLSCTASEFTFDDSDMAWYRQAPGNECELVSTITSDGGTYYADSVK
GRFTISRDNSKNTLYLQMNSLKPEDTAVYYCAADQWGSAEGDCTSSYPGGYWGQGTLVTVSS
(SEQ ID NO: 329)
> huFE148huv1 
QVQLVESGGGSVQAGGSLRLSCAASGYTFNNFVGWFRQAPGKEREGVAAIYSLGGSTYYADS
VKGRFTISQDNSKNTVYLQMNSLRAEDTAVYYCAGAITWVPPLSHRRYTYWGQGTLVTVSS
(SEQ ID NO: 330)
> huFE148huv2 
QVQLVESGGGLVQPGGSLRLSCAASGYTFNNNFVGWFRQAPGKGLEGVSAIYSLGGSTYYADSV
KGRFTISRDNSKNTLYLQMNSLRAEDTAVYYCAAAITWVPPLSHRRYTYWGQGTLVTVSS (SEQ
ID NO: 331)
> huFE148huv3 
QVQLVESGGGLVQPGGSLRLSCAASGYTFNNNFVGWFRQAPGKEREGVAAIYSLGGSTYYADS
VKGRFTISQDNSKNTVYLQMNSLRAEDTAVYYCAGAITWVPPLSHRRYTYWGQGTLVTVSS
(SEQ ID NO: 332)
> huFE148huv4 
QVQLVESGGGSVQAGGSLRLSCAASGYTFNNNFVGWFRQAPGKGLEGVSAIYSLGGSTYYADSV
KGRFTISQDNSKNTVYLQMNSLRAEDTAVYYCAGAITWVPPLSHRRYTYWGQGTLVTVSS (SEQ
ID NO: 333)
> huFE148huv5 
QVQLVESGGGSVQAGGSLRLSCAASGYTFNNNFVGWFRQAPGKGLEGVSAIYSLGGSTYYADSV
KGRFTISQDNSKNTLYLQMNSLKPEDTAVYYCAGAITWVPPLSHRRYTYWGQGTLVTVSS (SEQ
ID NO: 334)
> huFE148huv4 
QVQLVESGGGSVQAGGSLRLSCAASGYTFNNNFVGWFRQAPGKGLEGVSAIYSLGGSTYYADSV
KGRFTISRDNSKNTLYLQMNSLKPEDTAVYYCAGAITWVPPLSHRRYTYWGQGTLVTVSS (SEQ
ID NO: 335)
> IgG1-FC 
EPKSCDKTHTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 336)
> Apple1 
ECVTQLLKDTCFEGGDITTVFTPSAKYCQVVCTYHPRCLLFTFTAESPSEDPTRWFTCVLKDSVTET
LPRVNRTAAISGYSFKQCSHQISA (SEQ ID NO: 337)
> Apple2 
HQISACNKDIYVDLDMKGINYNSSVAKSAQECQERCTDDVHCHFFTYATRQFPSLEHRNICLLKHT
QTGTPTRITKLDKVVSGFSLKSCALSNLA (SEQ ID NO: 338)
> Apple3 
LSNLACIRDIFPNTVFADSNIDSVMAPDAFVCGRICTHHPGCLFFTFFSQEWPKESQRNLCLLKTSES
GLPSTRIKKSKALSGFSLQSCRHSIPVF (SEQ ID NO: 339)
> Apple4 
IPVFCHSSFYHDTDFLGEELDIVAAKSHEACQKLCTNAVRCQFFTYTPAQASCNEGKGKCYLKLSS
NGSPTKILHGRGGISGYTLRLCKMDNESTTK (SEQ ID NO: 340)
> Apple1-2 
ECVTQLLKDTCFEGGDITTVFTPSAKYCQVVCTYHPRCLLFTFTAESPSEDPTRWFTCVLKDSVTET
LPRVNRTAAISGYSFKQCSHQISACNKDIYVDLDMKGINYNSSVAKSAQECQERCTDDVHCHFFTY
ATRQFPSLEHRNICLLKHTQTGTPTRITKLDKVVSGFSLKSCALSNLA (SEQ ID NO: 341)
> Apple2-3 
HQISACNKDIYVDLDMKGINYNSSVAKSAQECQERCTDDVHCHFFTYATRQFPSLEHRNICLLKHT
QTGTPTRITKLDKVVSGFSLKSCALSNLACIRDIFPNTVFADSNIDSVMAPDAFVCGRICTHHPGCLF
FTFFSQEWPKESQRNLCLLKTSESGLPSTRIKKSKALSGFSLQSCRHSIPVF (SEQ ID NO: 342)
> Apple 3-4

HQISACNKDIYVDLDMKGINYNSSVAKSAQECQERCTDDVHCHFFTYATRQFPSLEHRNICLLKHT
QTGTPTRITKLDKVVSGFSLKSCALSNLACIRDIFPNTVFADSNIDSVMAPDAFVCGRICTHHPGCLF
FTFFSQEWPKESQRNLCLLKTSESGLPSTRIKKSKALSGFSLQSCRHSIPVF (SEQ ID NO: 343)

## Claims

1. A coagulation factor XI (FXI) binding protein, comprising at least one immunoglobulin single variable domain capable of specifically binding to FXI, the at least one immunoglobulin single variable domain comprising CDR1, CDR2 and CDR3 of a VHH set forth in any one of SEQ ID NOs: 4, 10 and 14.

2. The FXI binding protein according to claim 1, wherein the CDRs can be Kabat CDRs, AbM CDRs, Chothia CDRs or IMGT CDRs.

3. The FXI binding protein according to claim 1, wherein CDR1, CDR2 and CDR3 of the VHH set forth in SEQ ID NO: 4 are selected from any one of the following groups: SEQ ID NOs: 60-62, SEQ ID NOs: 63-65, SEQ ID NOs: 66-68 and SEQ ID NOs: 69-71.

4. The FXI binding protein according to claim 3, wherein the at least one immunoglobulin single variable domain comprises an amino acid sequence set forth in any one of SEQ ID NOs: 4, 306-311.

5. The FXI binding protein according to claim 1, wherein CDR1, CDR2 and CDR3 of the VHH set forth in SEQ ID NO: 10 are selected from any one of the following groups: SEQ ID NOs: 132-134, SEQ ID NOs: 135-137, SEQ ID NOs: 138-140 and SEQ ID NOs: 141-143.

6. The FXI binding protein according to claim 5, wherein the at least one immunoglobulin single variable domain comprises an amino acid sequence set forth in any one of SEQ ID NOs: 10, 312-317.

7. The FXI binding protein according to claim 1, wherein CDR1, CDR2 and CDR3 of the VHH set forth in SEQ ID NO: 14 are selected from any one of the following groups: SEQ ID NOs: 180-182, SEQ ID NOs: 183-185, SEQ ID NOs: 186-188 and SEQ ID NOs: 189-191.

8. The FXI binding protein according to claim 7, wherein the at least one immunoglobulin single variable domain comprises an amino acid sequence set forth in any one of SEQ ID NOs: 14, 318-328.

9. The FXI binding protein according to any one of claims 1 to 8, wherein the FXI binding protein binds to the Apple2 domain of FXI.

10. The FXI binding protein according to any one of claims 1 to 9, further comprising an immunoglobulin Fc region, preferably a human immunoglobulin Fc region, such as an Fc region of human IgG1, IgG2, IgG3 or IgG4.

11. The FXI binding protein according to claim 10, wherein an amino acid sequence of the immunoglobulin Fc region is set forth in SEQ ID NO: 336.

12. A coagulation factor XI (FXI) binding protein, comprising at least one immunoglobulin single variable domain capable of specifically binding to FXI, the at least one immunoglobulin single variable domain comprising CDR1, CDR2 and CDR3 of a VHH set forth in any one of SEQ ID NOs: 1, 17 and 20.

13. The FXI binding protein according to claim 12, wherein the CDRs can be Kabat CDRs, AbM CDRs, Chothia CDRs or IMGT CDRs.

14. The FXI binding protein according to claim 12, wherein CDR1, CDR2 and CDR3 of the VHH set forth in SEQ ID NO: 1 are selected from any one of the following groups: SEQ ID NOs: 24-26, SEQ ID NOs: 27-29, SEQ ID NOs: 30-32 and SEQ ID NOs: 33-35.

15. The FXI binding protein according to claim 14, wherein the at least one immunoglobulin single variable domain comprises an amino acid sequence set forth in any one of SEQ ID NOs: 1, 300-305.

16. The FXI binding protein according to claim 12, wherein CDR1, CDR2 and CDR3 of the VHH set forth in SEQ ID NO: 17 are selected from any one of the following groups: SEQ ID NOs: 216-218, SEQ ID NOs: 219-221, SEQ ID NOs: 222-224 and SEQ ID NOs: 225-227.

17. The FXI binding protein according to claim 16, wherein the at least one immunoglobulin single variable domain comprises an amino acid sequence set forth in any one of SEQ ID NOs: 17, 324-329.

18. The FXI binding protein according to claim 12, wherein CDR1, CDR2 and CDR3 of the VHH set forth in SEQ ID NO: 20 are selected from any one of the following groups: SEQ ID NOs: 252-254, SEQ ID NOs: 255-257, SEQ ID NOs: 258-260 and SEQ ID NOs: 261-263.

19. The FXI binding protein according to claim 18, wherein the at least one immunoglobulin single variable domain comprises an amino acid sequence set forth in any one of SEQ ID NOs: 20, 330-335.

20. The FXI binding protein according to any one of claims 1 to 19, wherein the FXI binding protein does not bind to the isolated Apple2 domain polypeptide of FXI.

21. The FXI binding protein according to any one of claims 1 to 20, further comprising an immunoglobulin Fc region, preferably a human immunoglobulin Fc region, such as an Fc region of human IgG1, IgG2, IgG3 or IgG4.

22. The FXI binding protein according to claim 21, wherein an amino acid sequence of the immunoglobulin Fc region is set forth in SEQ ID NO: 336.

23. A coagulation factor XI (FXI) binding protein, comprising a first immunoglobulin single variable domain capable of specifically binding to FXI and a second immunoglobulin single variable domain capable of specifically binding to FXI,
wherein the first immunoglobulin single variable domain and the second immunoglobulin single variable domain bind to different epitopes on FXI.

24. The FXI binding protein according to claim 23, wherein
the first immunoglobulin single variable domain binds to an epitope within the Apple2 domain of FXI, and the second immunoglobulin single variable domain binds to an epitope within the Apple3 domain of FXI; or
the first immunoglobulin single variable domain binds to an epitope within the Apple2 domain of FXI, and the second immunoglobulin single variable domain binds to an epitope within the Apple4 domain of FXI; or
the first immunoglobulin single variable domain binds to an epitope within the Apple2 domain of FXI, and the second immunoglobulin single variable domain binds to an epitope within the Apple2-3 region of FXI; or
the first immunoglobulin single variable domain binds to an epitope within the Apple3 domain of FXI, and the second immunoglobulin single variable domain binds to an epitope within the Apple4 domain of FXI; or
the first immunoglobulin single variable domain binds to an epitope within the Apple3 domain of FXI, and the second immunoglobulin single variable domain binds to an epitope within the Apple2-3 region of FXI; or
the first immunoglobulin single variable domain binds to an epitope within the Apple4 domain of FXI, and the second immunoglobulin single variable domain binds to an epitope within the Apple2-3 region of FXI.

25. The FXI binding protein according to claim 23 or 24, wherein
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 4; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 9; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 10; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 1, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 9; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 10; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 4, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 10; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 9, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 10, and the second immunoglobulin single variable domain comprises CDR1, CDR2 and CDR3 of a VHH set forth in SEQ ID NO: 14; or
the first immunoglobulin single variable domain comprises CDR1, CDR2 and CDR3 of a VHH set forth in SEQ ID NO: 10, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 10, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 14, and the second immunoglobulin single variable domain comprises CDR1, CDR2 and CDR3 of a VHH set forth in SEQ ID NO: 17; or
the first immunoglobulin single variable domain comprises CDR1, CDR2 and CDR3 of a VHH set forth in SEQ ID NO: 14, and the second immunoglobulin single variable domain comprises CDR1, CDR2 and CDR3 of a VHH set forth in SEQ ID NO: 20; or
the first immunoglobulin single variable domain comprises CDR1, CDR2 and CDR3 of a VHH set forth in SEQ ID NO: 17, and the second immunoglobulin single variable domain comprises CDR1, CDR2 and CDR3 of a VHH set forth in SEQ ID NO: 20.

26. The FXI binding protein according to claim 25, wherein the CDRs can be Kabat CDRs, AbM CDRs, Chothia CDRs or IMGT CDRs.

27. The FXI binding protein according to claim 25 or 26, wherein CDR1, CDR2 and CDR3 of the VHH set forth in SEQ ID NOs: 1, 4, 9, 10, 14, 17 or 20 are shown in the following table:
| **VHH sequence** | **CDR1-3 according to Kabat** | **CDR1-3 according to AbM** | **CDR1-3 according to Chothia** | **CDR1-3 according to IMGT** |
|---|---|---|---|---|
| **SEQ ID NO:1** | SEQ ID NO:24-26 | SEQ ID NO:27-29 | SEQ ID NO:30-32 | SEQ ID NO:33-35 |
| **SEQ ID NO:4** | SEQ ID NO:60-62 | SEQ ID NO:63-65 | SEQ ID NO:66-68 | SEQ ID NO:69-71 |
| **SEQ ID NO:9** | SEQ ID NO:120-122 | SEQ ID NO:123-125 | SEQ ID NO:126-128 | SEQ ID NO:129-131 |
| **SEQ ID NO:10** | SEQ ID NO:132-134 | SEQ ID NO:135-137 | SEQ ID NO:138-140 | SEQ ID NO:141-143 |
| **SEQ ID NO:14** | SEQ ID NO:180-182 | SEQ ID NO:183-185 | SEQ ID NO:186-188 | SEQ ID NO:189-191 |
| **SEQ ID NO:17** | SEQ ID NO:216-218 | SEQ ID NO:219-221 | SEQ ID NO:222-224 | SEQ ID NO:225-227 |
| **SEQ ID NO:20** | SEQ ID NO:252-254 | SEQ ID NO:255-257 | SEQ ID NO:258-260 | SEQ ID NO:261-263 |

28. The FXI binding protein according to any one of claims 23 to 27, comprising the first immunoglobulin single variable domain and the second immunoglobulin single variable domain, wherein
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 4, 306-311; or
the first immunoglobulin single variable domain comprises an amino acid sequence set forth in any one of SEQ ID NOs: 1, 300-305, and the second immunoglobulin single variable domain comprises an amino acid sequence set forth in SEQ ID NO: 9; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 10, 312-317; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 14, 318-323; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 17, 324-329; or
the first immunoglobulin single variable domain comprises an amino acid sequence set forth in any one of SEQ ID NOs: 1, 300-305, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 4, 306-311, and the second immunoglobulin single variable domain comprises an amino acid sequence set forth in SEQ ID NO: 9; or
the first immunoglobulin single variable domain comprises an amino acid sequence set forth in any one of SEQ ID NOs: 4, 306-311, and the second immunoglobulin single variable domain comprises an amino acid sequence of a VHH set forth in any one of SEQ ID NOs: 10, 312-317; or
the first immunoglobulin single variable domain comprises an amino acid sequence set forth in any one of SEQ ID NOs: 4, 306-311, and the second immunoglobulin single variable domain comprises an amino acid sequence set forth in any one of SEQ ID NOs: 14, 318-323; or
the first immunoglobulin single variable domain comprises an amino acid sequence set forth in any one of SEQ ID NOs: 4, 306-311, and the second immunoglobulin single variable domain comprises an amino acid sequence set forth in any one of SEQ ID NOs: 17, 324-329; or
the first immunoglobulin single variable domain comprises an amino acid sequence set forth in any one of SEQ ID NOs: 4, 306-311, and the second immunoglobulin single variable domain comprises an amino acid sequence set forth in any one of SEQ ID NOs: 20, 330-335; or
the first immunoglobulin single variable domain comprises an amino acid sequence set forth in SEQ ID NO: 9, and the second immunoglobulin single variable domain comprises an amino acid sequence set forth in any one of SEQ ID NOs: 10, 312-317; or
the first immunoglobulin single variable domain comprises an amino acid sequence set forth in SEQ ID NO: 9, and the second immunoglobulin single variable domain comprises an amino acid sequence set forth in any one of SEQ ID NOs: 14, 318-323; or
the first immunoglobulin single variable domain comprises an amino acid sequence set forth in SEQ ID NO: 9, and the second immunoglobulin single variable domain comprises an amino acid sequence set forth in any one of SEQ ID NOs: 17, 324-329; or
the first immunoglobulin single variable domain comprises an amino acid sequence set forth in SEQ ID NO: 9, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 10, 312-317, and the second immunoglobulin single variable domain comprises an amino acid sequence set forth in any one of SEQ ID NOs: 14, 318-323; or
the first immunoglobulin single variable domain comprises an amino acid sequence set forth in any one of SEQ ID NOs: 10, 312-317, and the second immunoglobulin single variable domain comprises an amino acid sequence set forth in any one of SEQ ID NOs: 17, 324-329; or
the first immunoglobulin single variable domain comprises an amino acid sequence set forth in any one of SEQ ID NOs: 10, 312-317, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 14, 318-323, and the second immunoglobulin single variable domain comprises an amino acid sequence set forth in any one of SEQ ID NOs: 17, 324-329; or
the first immunoglobulin single variable domain comprises an amino acid sequence set forth in any one of SEQ ID NOs: 14, 318-323, and the second immunoglobulin single variable domain comprises an amino acid sequence set forth in any one of SEQ ID NOs: 20, 330-335; or
the first immunoglobulin single variable domain comprises an amino acid sequence set forth in any one of SEQ ID NOs: 17, 324-329, and the second immunoglobulin single variable domain comprises an amino acid sequence set forth in any one of SEQ ID NOs: 20, 330-335.

29. The FXI binding protein according to any one of claims 23 to 28, wherein the first immunoglobulin single variable domain is located at the N-terminus of the second immunoglobulin single variable domain; or wherein the second immunoglobulin single variable domain is located at the N-terminus of the first immunoglobulin single variable domain.

30. The FXI binding protein according to any one of claims 23 to 29, further comprising an immunoglobulin Fc region, preferably a human immunoglobulin Fc region, such as an Fc region of human IgG1, IgG2, IgG3 or IgG4.

31. The FXI binding protein according to claim 30, wherein an amino acid sequence of the immunoglobulin Fc region is set forth in SEQ ID NO: 336.

32. A nucleic acid molecule encoding the FXI binding protein according to any one of claims 1 to 31.

33. An expression vector, comprising the nucleic acid molecule according to claim 32 operably linked to an expression control element.

34. A recombinant cell comprising the nucleic acid molecule according to claim 32 or transformed with the expression vector according to claim 33 and capable of expressing the FXI binding protein.

35. A method for producing the FXI binding protein according to any one of claims 1 to 31, comprising:
a) culturing the recombinant cell according to claim 34 under a condition that the FXI binding protein is allowed to be expressed;
b) recovering the FXI binding protein expressed by the recombinant cell from the culture of the step a); and
c) optionally further purifying and/or modifying the FXI binding protein of the step b).

36. A pharmaceutical composition, comprising the FXI binding protein according to any one of claims 1 to 31 and a pharmaceutically acceptable carrier.

37. A method for treating and/or preventing a thromboembolic condition or disease in a subject, comprising administering to the subject a therapeutically effective amount of the FXI binding protein according to any one of claims 1 to 31 or the pharmaceutical composition according to claim 36.

38. The method according to claim 37, wherein the subject has or is at risk of myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, arterial thrombosis, advanced renal disease, antiphospholipid syndrome, cerebralvascular accident, metastatic cancer or infectious disease.

39. Use of the FXI binding protein according to any one of claims 1 to 31 or the pharmaceutical composition according to claim 36 in the manufacture of a medicament for the treatment and/or prevention of a thromboembolic condition or disease.

40. The use according to claim 39, wherein the thromboembolic condition or disease is myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, thromboembolism formed in extracorporeal circulation (such as cardiopulmonary bypass, hemodialysis and ECMO), arterial thrombosis, advanced renal disease, antiphospholipid syndrome, cerebralvascular accident, metastatic cancer or infectious disease.

41. A method for inhibiting an activation of FXI in a subject, comprising: (a) selecting a subject in need of treatment, wherein the subject in need of treatment has or is at risk of thrombosis; and (b) administering to the subject an effective amount of the FXI binding protein according to any one of claims 1 to 31 or the pharmaceutical composition according to claim 36, thereby inhibiting the activation of FXI.

42. The method according to claim 41, wherein the subject in need of treatment is a subject having or at risk of myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, thromboembolism formed in extracorporeal circulation (such as cardiopulmonary bypass, hemodialysis and ECMO), arterial thrombosis, advanced renal disease, antiphospholipid syndrome, cerebralvascular accident, metastatic cancer or infectious disease.

43. A method for inhibiting coagulation and related thrombosis in a subject in need thereof without compromising hemostasis, comprising administering to the subject a therapeutically effective amount of the FXI binding protein according to any one of claims 1 to 31 or the pharmaceutical composition according to claim 36, thereby inhibiting coagulation and related thrombosis in the subject without compromising hemostasis.

44. The method according to claim 43, wherein the subject has or is at risk of myocardial infarction, ischemic stroke, pulmonary thromboembolism, venous thromboembolism (VTE), atrial fibrillation, disseminated intravascular coagulation, medical device-related thromboembolic condition, severe systemic inflammatory response syndrome, thromboembolism formed in extracorporeal circulation (such as cardiopulmonary bypass, hemodialysis and ECMO), arterial thrombosis, advanced renal disease, antiphospholipid syndrome, cerebralvascular accident, metastatic cancer or infectious disease.

45. Use of the FXI binding protein according to any one of claims 1 to 31 or the pharmaceutical composition according to claim 36 in the manufacture of a medicament for the inhibition of coagulation and related thrombosis without compromising hemostasis.
